(19) Europäisches Patentamt
European Patent Office
Office européen des brevets

(11) Publication number: 0 394 464 A1

(12) EUROPEAN PATENT APPLICATION
published in accordance with Art. 158(3) EPC

(21) Application number: 89909435.3

(22) Date of filing: 21.08.89

(86) International application number:
PCT/JP89/00847

(87) International publication number:
WO 90/02125 (08.03.90 90/06)

(51) Int. Cl.[5]: C07D 405/12, C07D 413/12, C07D 417/12

(30) Priority: 22.08.88 JP 206372/88
27.02.89 JP 143316/89
27.02.89 JP 143317/89
11.07.89 JP 117256/89

(43) Date of publication of application:
31.10.90 Bulletin 90/44

(84) Designated Contracting States:
BE CH DE FR GB IT LI NL SE

(71) Applicant: IDEMITSU KOSAN COMPANY LIMITED
1-1, Marunouchi 3-chome Chiyoda-ku
Tokyo 100(JP)

(72) Inventor: NISHII, Masahiro Idemitsu Kosan Company Limited
1280, Kamiizumi, Sodegauramachi
Kimitsu-gun Chiba 299-02(JP)
Inventor: TAKAHASHI, Kazuyuki Idemitsu Kosan Company Ltd.
1280, Kamiizumi, Sodegauramachi,
Kimitsu-gun Chiba 299-02(JP)
Inventor: HIROI, Yoshio Idemitsu Kosan Company Limited
1280, Kamiizumi Sodegauramachi,
Kimitsu-gun Chiba 299-02(JP)
Inventor: HIRATA, Toshihiro Idemitsu Kosan Company Limited
1280, Kamiizumi Sodegauramachi,
Kimitsu-gun Chiba 299-02(JP)

(74) Representative: Türk, Gille, Hrabal
Brucknerstrasse 20
D-4000 Düsseldorf 13(DE)

(54) OXIRANE DERIVATIVES AND HERBICIDES CONTAINING SAME AS ACTIVE INGREDIENTS.

EP 0 394 464 A1

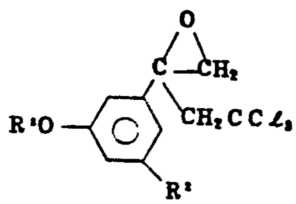

··· (III)

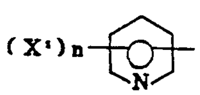 (a) 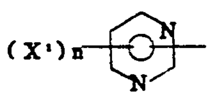 (b) 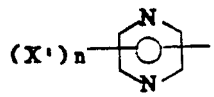 (c) 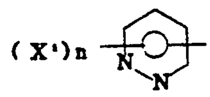 (d)

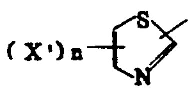 (e) 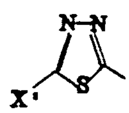 (f) 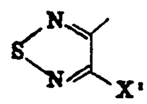 (g) 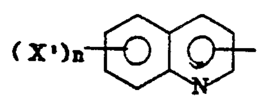 (h)

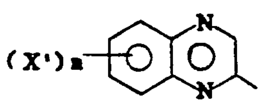 (i) 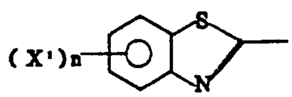 (j) 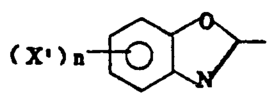 (k)

(57) Oxirane derivatives represented by general formula (III) (wherein $R^1$ represents a, b, c, d, e, f, g, h, i, j or k, $X^1$ represents a halogen atom, an alkyl group, a haloalkyl group, an alkoxyl group, a nitro group, a cyano group, an amide group, an acylamino group or an alkylsulfonyl group, and n represents an integer of 0 to 4, provided that, when n represents 2 or more, $X^1$'s may be the same or different from each other, and $R^2$ represents a hydrogen atom or an alkoxyl group) and herbicides containing the oxirane derivatives represented by general formula (III) as active ingredients.

S P E C I F I C A T I O N

# AN OXIRANE DERIVATIVE AND A HERBICIDE CONTAINING THE SAME AS AN EFFECTIVE INGREDIENT

## Field of Technology

The present invention relates to a novel oxirane derivative and a herbicide containing the same as an effective ingredient.

## Background Technology

Herbicides are very important chemicals for labor saving in the weed-controlling works and improvement of the productivity of agricultural and horticultural crops so that research and development have been actively undertaken over long years and a large variety of chemicals have now been rendered to practical use but it is desired still in these days to develop a novel chemical having a prominent herbicidal characteristics or, in particular, a chemical capable of controlling the objective species of weed alone selectively and in a low dose without giving damages by the chemicals to the crops under growing.

It is known, on the other hand, that various kinds of weeds such as, for example, annual weeds belonging to the family of Gramineae, e.g., Echinochloa crus-galli P. Beauv. var. formosensis Ohwi and the like, annual weeds belonging

to the family of Zingiberaceae, e.g., Cyperus difformis L. and the like, annual broadleaf weeds, e.g., Monochoria vaginalis Presl var. plantaginea, Rotala indica (Willd.) Koehne var. uliginosa (Miq.) Koehne and the like, and perennial weeds, e.g., Sagittaria pygmaea Miq., Potamogeton distinctus A. Bennett, Alisma canaliculatum A Br. et Bouche, Scirpus juncoides Roxb. ssp. Hotarui Ohwi T. Koyama, Eleocharis acicularis Roem. et Schult var. longiseta Svenson, Cyperus serotinus Rottb., Eleocharis kuroguwai Ohwi, Sagittaria trifolia L., Oenanthe javanica (Blume) DC. and the like, grow in paddy fields together with paddy rice and it is very important for rice cropping to remove these weeds efficiently without damaging the paddy rice with chemicals and by scattering a small amount in view of the problem of environmental contamination. Since Echinochloa crus-galli P. Beauv. var. formosensis Ohwi is a weed belonging to the family of Gramineaea, in particular, chemicals having herbicidal activity against Echinochloa crus-galli P. Beauv. var. formosensis Ohwi are liable to chemical damages against paddy rice so that it is an important problem to develop a chemical having high herbicidal activity against Echinochloa crus-galli P. Beauv. var. formosensis Ohwi and excellent in the inter-genus selectivity between paddy rice and Echinochloa crus-galli P. Beauv. var. formosensis Ohwi.

Known oxirane derivatives having herbicidal activity include, for example, a compound expressed by the formula

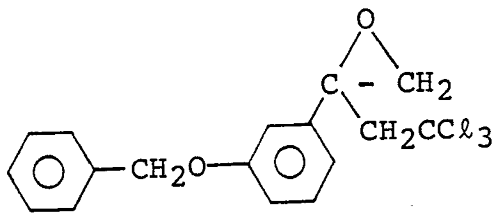

.... (I)

and a compound expressed by the formula

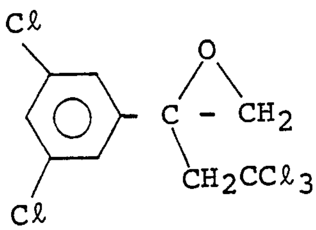

... (II)

[trivial name "tridiphane", name of commercial product by Dow Chemical Co. "Tandem"].

Though effective by a treatment before germination against the weeds belonging to the family of Gramineae such as big weed, crab grass, Johnson-grass, wild oat, yellow foxtail and the like, however, the compound expressed by the above given formula (I) has only low herbicidal activity and no actual case of the use thereof for paddy rice has yet been reported. On the other hand, the compound expressed by the above given formula (II) has already been rendered to practical use for Indian corn fields as a herbicide for the control of weeds of family Gramineae but, when it is used as a herbicide for paddy rice, the paddy rice may be subject to chemical damage due to the poor selectivity.

An object of the present invention is to provide a novel compound having excellent herbicidal activity against weeds in paddy fields and capable of efficiently removing

the weeds in paddy fields in a low dose by keeping the chemical damage against paddy rice very low as well as a herbicide containing the same as an effective ingredient.

The inventors, directing their attention to the above mentioned oxirane derivatives having herbicidal activity against the plants of family Gramineae, have continued extensive investigations on an oxirane derivative having excellent herbicidal activity and selectivity against not only annual weeds of family Gramineae such as Echinochloa crus-galli P. Beauv. var. formosensis Ohwi and the like but also other weeds in paddy fields and, as a result, have arrived at a discovery that a novel oxirane derivative having a specific nitrogen-containing aryloxyphenyl skeleton has excellent herbicidal activity against Echinochloa crus-galli P. Beauv. var. formosensis Ohwi, Cyperus difformis L., Cyperus serotinus Rottb., annual broadleaf weeds and the like along with high selectivity or, in particular, excellent inter-genus selectivity between paddy rice and Echinochloa crus-galli P. Beauv. var. formosensis Ohwi so as to be capable of efficiently removing the weeds in paddy fields with almost no chemical damage against paddy rice leading to completion of the present invention on the base of this discovery.

Disclosure of the Invention

The present invention provides (1) an oxirane derivative represented by the general formula

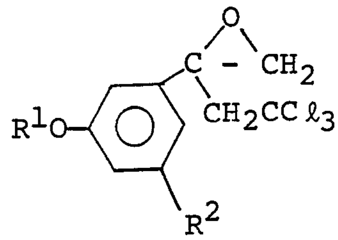

(in the formula, $R^1$ is

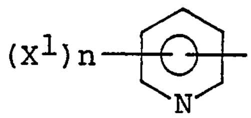 , 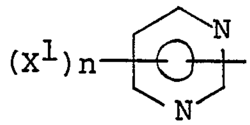 , 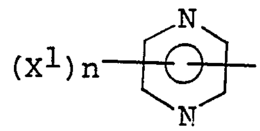 ,

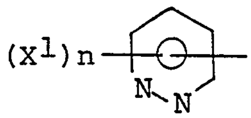 , 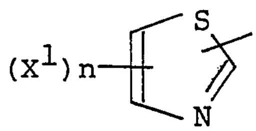 , 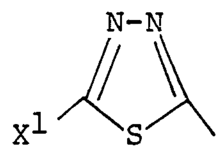 ,

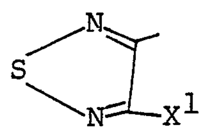 , 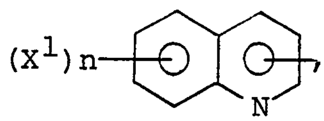 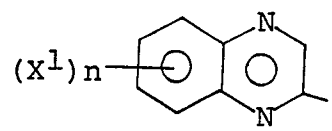 ,

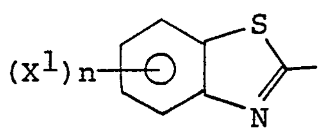 or 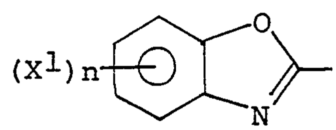 ,

$X^1$ is a halogen atom, alkyl group having 1 to 4 carbon atoms, haloalkyl group having 1 to 4 carbon atoms, alkoxy group having 1 to 4 carbon atoms, nitro group, cyano group, acid amide group, acylamino group having 1 to 4 carbon atoms or alkylsulfonyl group having 1 to 4 carbon atoms, n is an integer of 0 to 4 and $R^2$ is a hydrogen atom or an alkoxy group having 1 to 4 carbon atoms, each $X^1$ being the same as or different from the others when n is a plural number), and

(2) a herbicide containing, as an effective ingredient, an oxirane derivative represented by the general formula

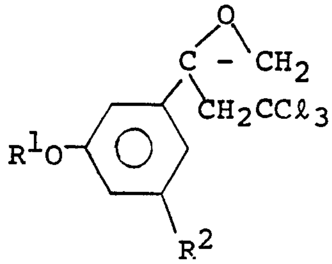

(in the formula, $R^1$ is

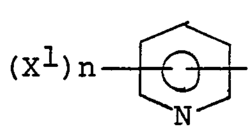 , 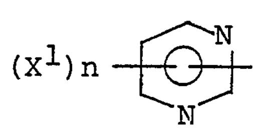 , 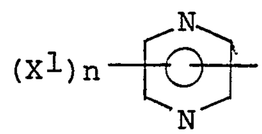 ,

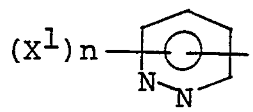 , 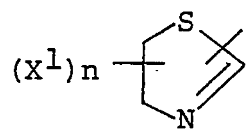 , 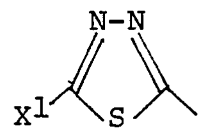 ,

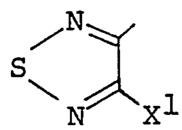 , 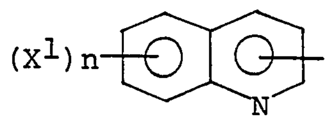 , 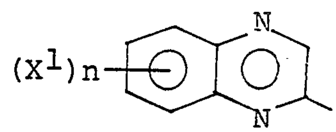 ,

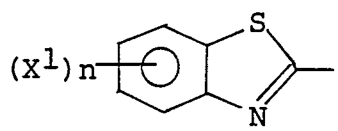 or 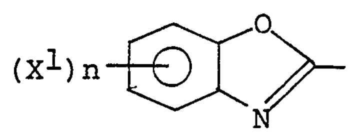 ,

$X^1$ is a halogen atom, alkyl group having 1 to 4 carbon atoms, haloalkyl group having 1 to 4 carbon atoms, alkoxy group having 1 to 4 carbon atoms, nitro group, cyano group, acid amide group, acylamino group having 1 to 4 carbon atoms or alkylsulfonyl group having 1 to 4 carbon atoms, n is an integer of 0 to 4 and $R^2$ is a hydrogen atom or an alkoxy group having 1 to 4 carbon atoms, each $X^1$ being the same as or different from the others when n is a plural number).

The oxirane derivative of the present invention is a novel compound having a nitrogen-containing aryloxyphenyl skeleton and can be used effectively as a herbicide. Further, the herbicide of the present invention has excellent herbicidal activity against Echinochloa crus-galli P. Beauv. var.

formosensis Ohwi, Cyperus difformis L., Cyperus serotinus Rottb., annual broadleaf weeds and the like as the weeds in paddy fields along with high selectivity or, in particular, excellent inter-genus selectivity between paddy rice and Echinochloa crus-galli P. Beauv. var. formosensis Ohwi and is capable of effectively controlling the above mentioned weeds starting with Echinochloa crus-galli P. Beauv. var. formosensis Ohwi at a low dose giving almost no chemical damages against paddy rice.

## Best Mode to Practice the Invention

The present invention provides an oxirane derivative represented by the general formula

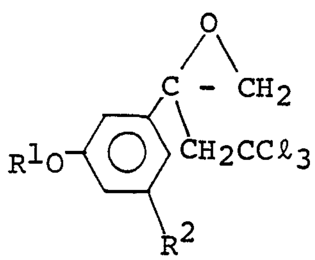

.... (III)

(in the formula, $R^1$ is

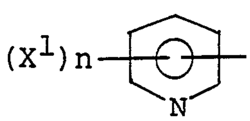 , 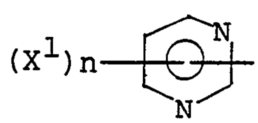 , 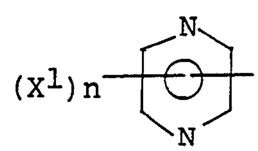 ,

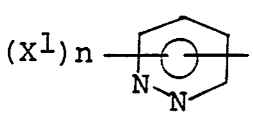 , 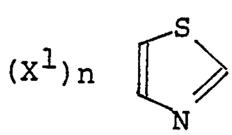 , 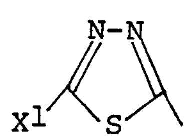 ,

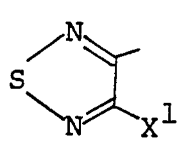 , 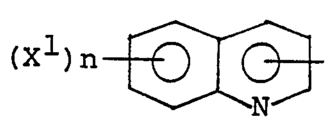 , 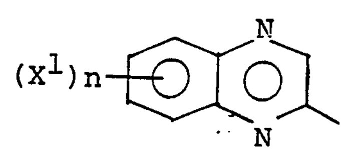 ,

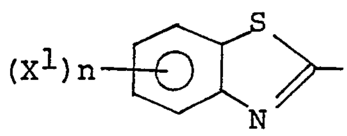 or 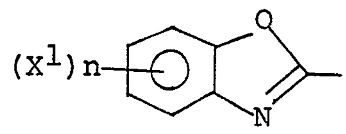

$X^1$ is a halogen atom, alkyl group having 1 to 4 carbon atoms, haloalkyl group having 1 to 4 carbon atoms, alkoxy group having 1 to 4 carbon atoms, nitro group, cyano group, acid amide group, acylamino group having 1 to 4 carbon atoms or alkylsulfonyl group having 1 to 4 carbon atoms, n is an integer of 0 to 4 and $R^2$ is a hydrogen atom or alkoxy group having 1 to 4 carbon atoms, each $X^1$ being the same as or different from the others when n is a plural number) as well as a herbicide containing the oxirane derivative represented by the general formula III as an effective ingredient.

The compound represented by the above given general formula III is a novel compound. In the formula, $R^1$ is

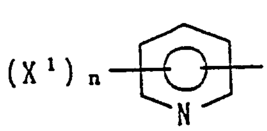, 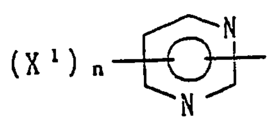, 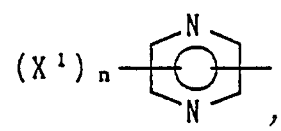,

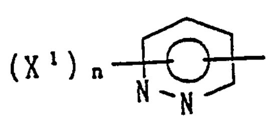, 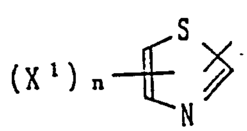, 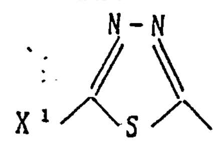,

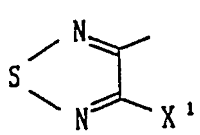, 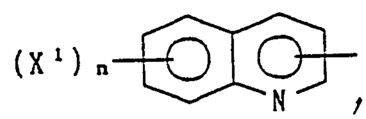, 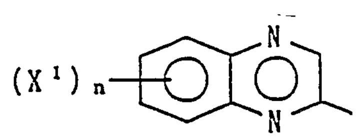

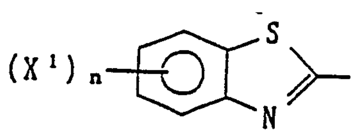 or 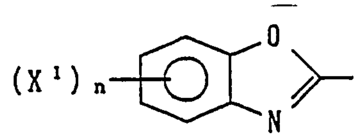

$X^1$ is a halogen atom, alkyl group having 1 to 4 carbon atoms, haloalkyl group having 1 to 4 carbon atoms, alkoxy group having

1 to 4 carbon atoms, nitro group, cyano group, acid amide group, acylamino group having 1 to 4 carbon atoms or alkylsulfonyl group having 1 to 4 carbon atoms and n is an integer of 0 to 4. Incidentally, each $X^1$ can be the same as or different from the others when n is a plural number. And, $R^2$ is a hydrogen atom or an alkoxy group having 1 to 4 carbon atoms.

Typical examples of the oxirane derivative of the present invention include: 2-[3-(5-trifluoromethyl-2-pyridyloxy)phenyl]-2-(2,2,2-trichloroethyl) oxirane (compound No. 1); 2-[3-(6-trifluoromethyl-2-pyridyloxy)phenyl]-2-(2,2,2-trichloroethyl) oxirane (compound No. 2); 2-[3-(5-trifluoromethyl-6-chloro-2-pyridyloxy)phenyl]-2-(2,2,2-trichloroethyl) oxirane (compound No. 3); 2-[3-(5-chloro-2-pyridyloxy)phenyl]-2-(2,2,2-trichloroethyl) oxirane (compound No. 4); 2-[3-(2-pydidyloxy)phenyl]-2-(2,2,2-trichloroethyl) oxirane (compound No. 5); 2-[3-(6-chloro-2-pyridyloxy)phenyl]-2-(2,2,2-trichloroethyl) oxirane (compound No. 6); 2-[3-(6-bromo-2-pyridyloxy)phenyl]-2-(2,2,2-trichloroethyl) oxirane (compound No. 7); 2-[3-(6-methoxy-2-pyridyloxy)phenyl]-2-(2,2,2-trichloroethyl) oxirane (compound No. 8); 2-[3-(5-bromo-2-pyridyloxy)phenyl]-2-(2,2,2-trichloroethyl) oxirane (compound No. 9); 2-[3-(6-methyl-2-pyridyloxy)-phenyl]-2-(2,2,2-trichloroethyl) oxirane (compound No. 10); 2-[3-(5-nitro-2-pyridyloxy)phenyl]-2-(2,2,2-trichloroethyl) oxirane (compound No. 11); 2-[3-(6-fluoro-2-pyridyloxy)phenyl]-2-(2,2,2-trichloroethyl) oxirane (compound No. 12); 2-[3-(6-ethoxy-2-pyridyloxy)phenyl]-2-(2,2,2-trichloroethyl) oxirane (compound No. 13); 2-[3-(5-methyl-2-pyridyloxy)phenyl]-2-(2,2,2-trichloroethyl) oxirane (compound No. 14); 2-[3-(3,5,6-trifluoro-2-pyridyloxy)phenyl]-2-(2,2,2-trichloroethyl) oxirane (compound No. 15); 2-[3-(5-trifluoromethyl-2-pyridyloxy)-5-methoxyphenyl]-2-(2,2,2-trichloroethyl) oxirane (compound No. 16); 2-[3-(3,5,6-trifluoro-4-methylpyridyloxy)phenyl]-2-(2,2,2-trichloroethyl) oxirane (compound No. 17); 2-[3-(4-trifluoromethyl-2-pyridyloxy)phenyl]-2-(2,2,2-trichloroethyl) oxirane (compound No. 18); 2-[3-(4-aminocarboxy-2-pyridyloxy)phenyl]-2-(2,2,2-trichloroethyl) oxirane (compound No. 19); 2-[3-(3-chloro-2-pyridyloxy)-phenyl]-2-(2,2,2-trichloroethyl) oxirane (compound No. 20); 2-[3-(5-cyano-2-pyridyloxy)phenyl]-2-(2,2,2-trichloroethyl) oxirane (compound No. 21); 2-[3-(6-methylsulfonyl-2-pyridyloxy)-phenyl]-2-(2,2,2-trichloroethyl) oxirane (compound No. 22); 2-[3-(3-fluoro-2-pyridyloxy)phenyl]-2-(2,2,2-trichloroethyl) oxirane (compound No. 23); 2-[3-(5-bromomethyl-2-pyridyloxy)-phenyl]-2-(2,2,2-trichloroethyl) oxirane (compound No. 24); 2-[3-(5-fluoromethyl-2-pyridyloxy)phenyl]-2-(2,2,2-trichloroethyl) oxirane (compound No. 25); 2-[3-(5-difluoromethyl-2-pyridyloxy)phenyl]-2-(2,2,2-trichloroethyl) oxirane (compound No. 26); 2-[3-(5-fluoro-2-pyridyloxy)phenyl]-2-(2,2,2-trichloroethyl) oxirane (compound No. 27); 2-[3-(5-acetylamino-2-pyridyloxy)phenyl]-2-(2,2,2-trichloroethyl) oxirane (compound No. 28), 2-[3-(thiazol-2-yloxy)phenyl]-2-(2,2,2-trichloroethyl) oxirane (compound No. 29); 2-[3-(benzothiazol-2-yloxy)phenyl]-2-(2,2,2-trichloroethyl) oxirane (compound No. 30); 2-[3-(pyrimidin-2-yloxy)phenyl]-2-(2,2,2-trichloroethyl) oxirane (compound No. 31); 2-[3-(6-chloropyrimidin-4-yloxy)phenyl]-2-(2,2,2-trichloroethyl) oxirane (compound No. 32); 2-[3-(2-chloropyrimidin-4-yloxy)phenyl]-2-(2,2,2-trichloroethyl) oxirane (compound

No. 33); 2-[3-(5-bromopyrimidin-2-yloxy)phenyl]-2-(2,2,2-trichloroethyl) oxirane (compound No. 34); 2-[3-(5-methylpyrimidin-2-yloxy)phenyl]-2-(2,2,2-trichloroethyl) oxirane (compound No. 35); 2-[3-(pyrazin-2-yloxy)phenyl]-2-(2,2,2-trichloroethyl) oxirane (compound No. 36); 2-[3-(6-chloropyrazin-2-yloxy)phenyl]-2-(2,2,2-trichloroethyl) oxirane (compound No. 37); 2-[3-(6-chloropyridazin-3-yloxy)phenyl]2-(2,2,2-trichloroethyl) oxirane (compound No. 38); 2-[3-(benzoxazol-2-yloxy)phenyl]-2-(2,2,2-trichloroethyl) oxirane (compound No. 39); 2-[3-(5-chlorothiazol-2-yloxy)phenyl]-2-(2,2,2-trichloroethyl) oxirane (compound No. 40); 2-[3-(5-chloropyrazin-2-yloxy)phenyl]-2-(2,2,2-trichloroethyl) oxirane (compound No. 41); 2-[3-(quinoxalin-2-yloxy)phenyl]-2-(2,2,2-trichloroethyl) oxirane (compound No. 42); 2-[3-(quinolin-2-yloxy)phenyl]-2-(2,2,2-trichloroethyl) oxirane (compound No. 43); 2-[3-(3-chloropyrazin-2-yloxy)phenyl]-2-(2,2,2-trichloroethyl) oxirane (compound No. 44); 2-[3-(4-methylthiazol-2-yloxy)phenyl]-2-(2,2,2-trichloroethyl) oxirane (compound No. 45); 2-[3-(6-fluoropyrazin-2-yloxy)phenyl]-2-(2,2,2-trichloroethyl) oxirane (compound No. 46); 2-[3-(4-chloro-1,2,5-thiadiazol-3-yloxy)phenyl]-2-(2,2,2-trichloroethyl) oxirane (compound No. 47); 2-[3-(6-fluoropyridazin-3-yloxy)phenyl]-2-(2,2,2-trichloroethyl) oxirane (compound No. 48); 2-[3-(5-trifluoromethyl-1,3,4-thiadiazol-2-yloxy)phenyl]-2-(2,2,2-trichloroethyl) oxirane (compound No. 49); 2-[3-(5-fluoropyrazin-2-yloxy)phenyl]-2-(2,2,2-trichloroethyl) oxirane (compound No. 50); 2-[3-(3-fluoropyrazin-2-yloxy)phenyl]-2-(2,2,2-trichloroethyl) oxirane (compound No. 51); 2-[3-(5-methyl-1,3,4-thiadiazol-2-yloxy)phenyl]-2-(2,2,2-trichloroethyl) oxirane

(compound No. 52); 2-[3-(4-chlorothiazol-2-yloxy)phenyl]-2-(2,2,2-trichloroethyl) oxirane (compound No. 53) and the like though not limited to these compounds only.

In the following, the structural formulas are shown of the above named oxirane derivatives.

compound No.1

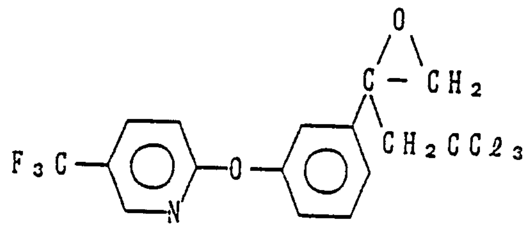

compound No. 2

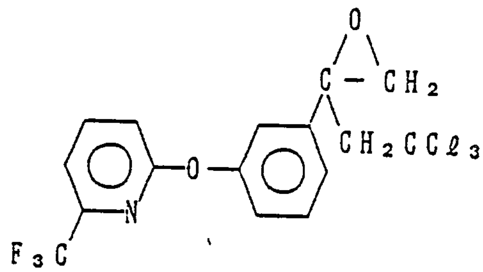

compound No. 3

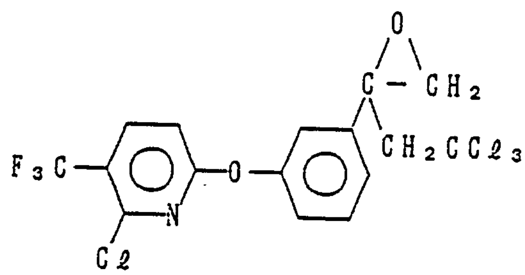

compound No. 4

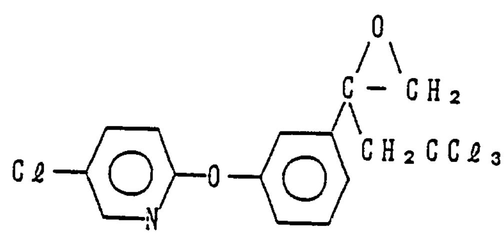

compound No. 5

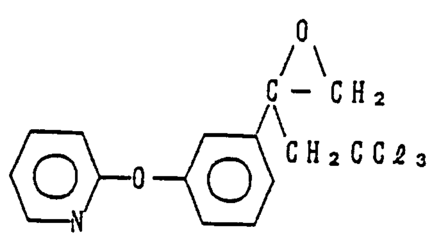

compound No. 6

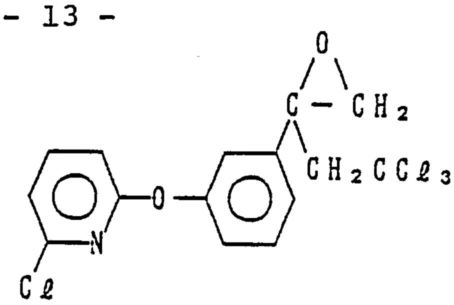

compound No. 7

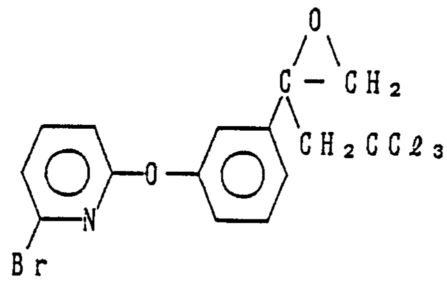

compound No. 8

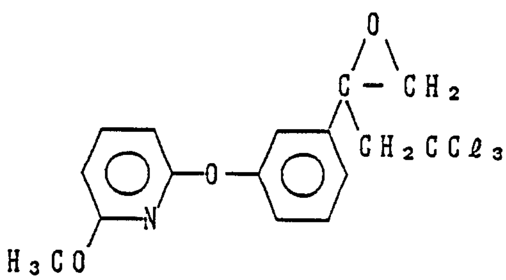

compound No. 9

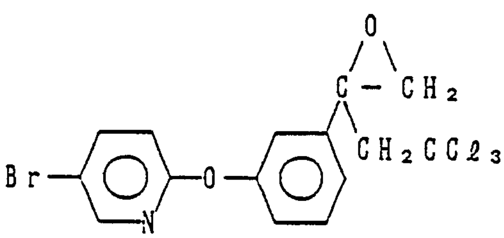

compound No. 10

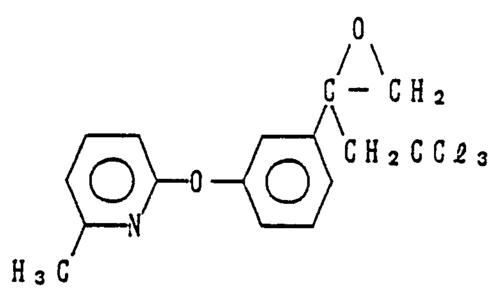

compound No. 11

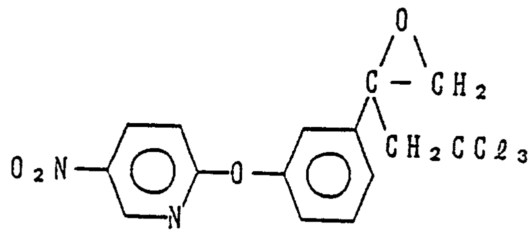

compound No. 12

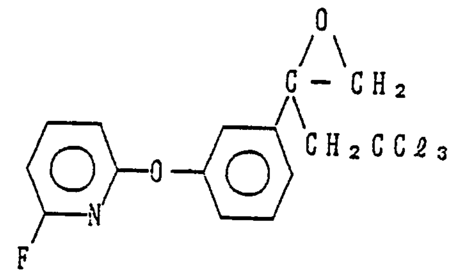

compound No. 13

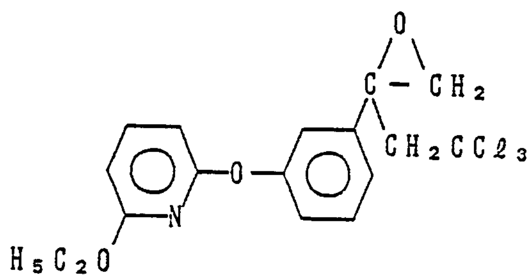

compound No. 14

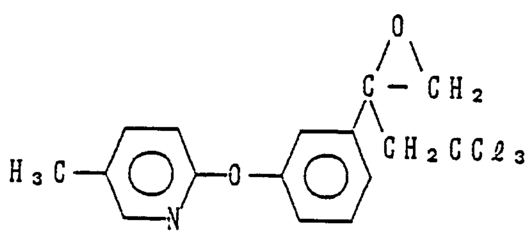

compound No. 15

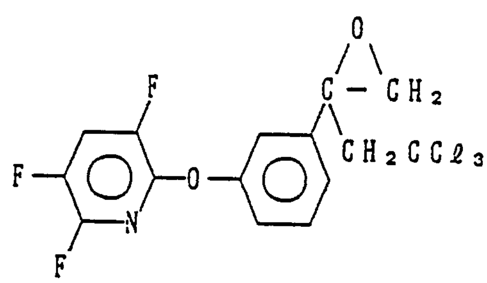

compound No. 16

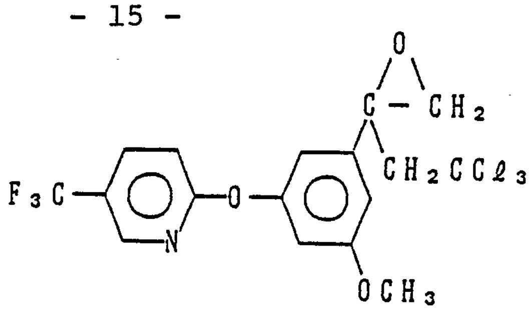

compound No. 17

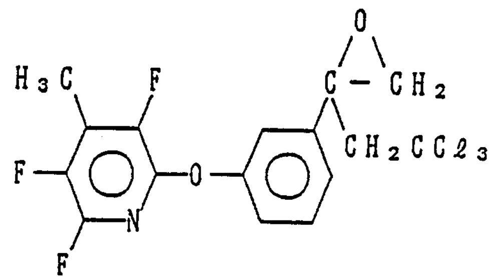

compound No. 18

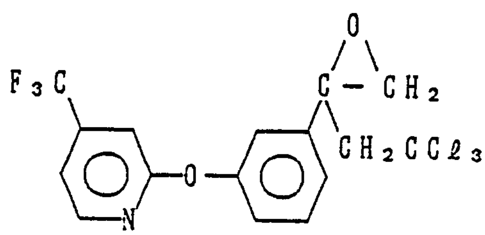

compound No. 19

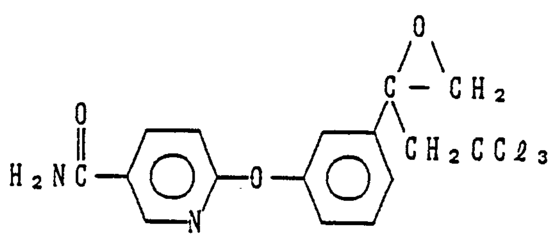

compound No. 20

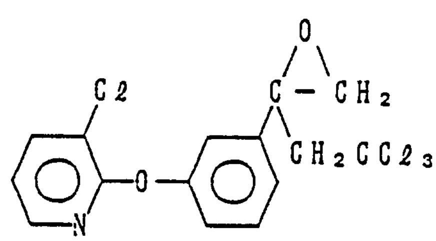

compound No. 21

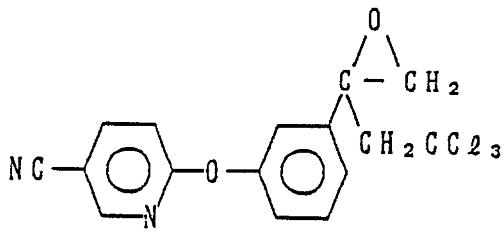

compound No. 22

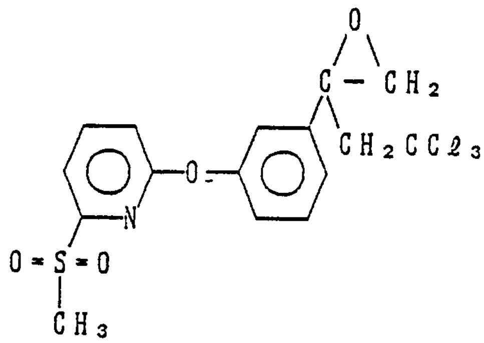

compound No. 23

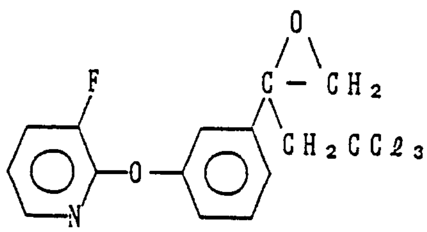

compound No. 24

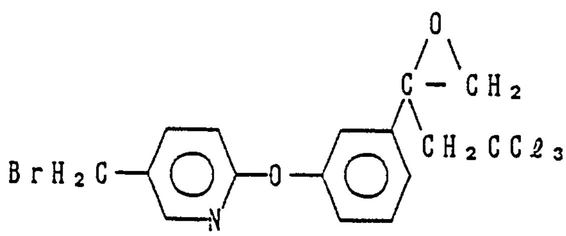

compound No. 25

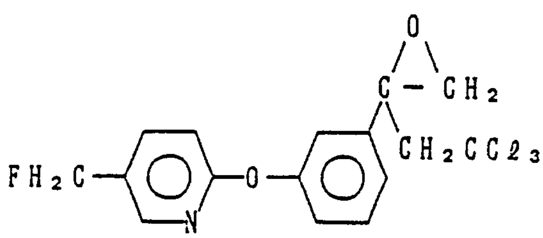

compound No. 26

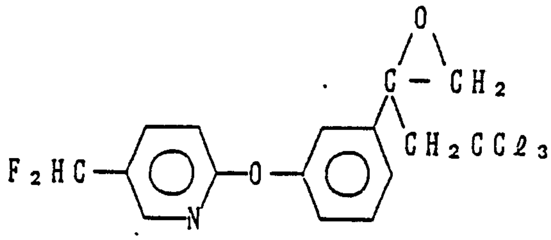

compound No. 27

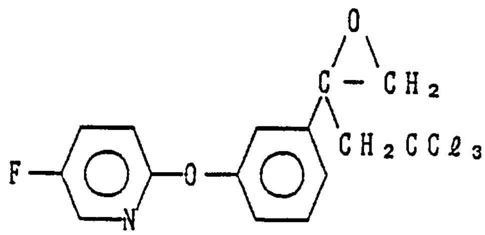

compound No. 28

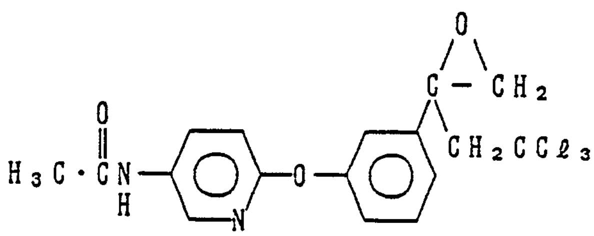

compound No. 29

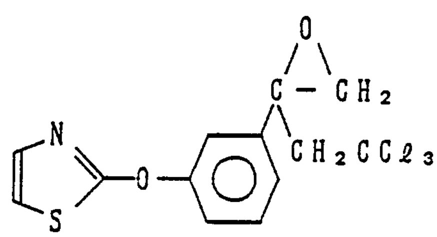

compound No. 30

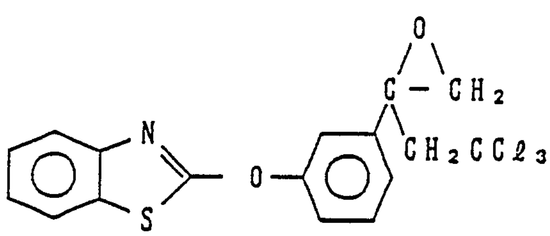

compound No. 31

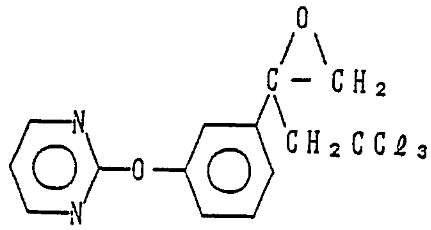

compound No. 32

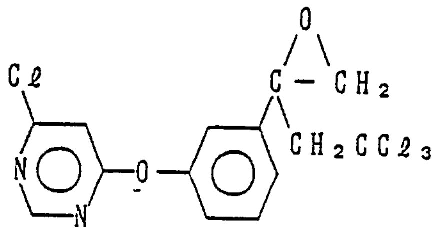

compound No. 33

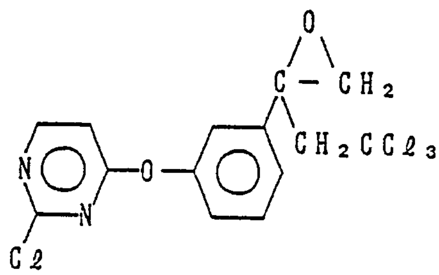

compound No. 34

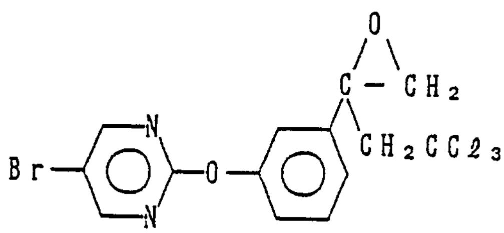

compound No. 35

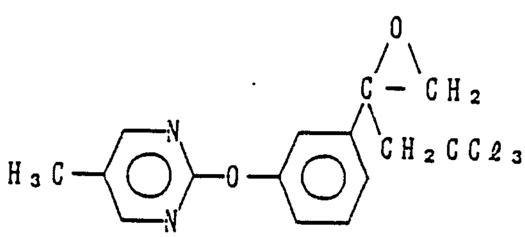

compound No. 36

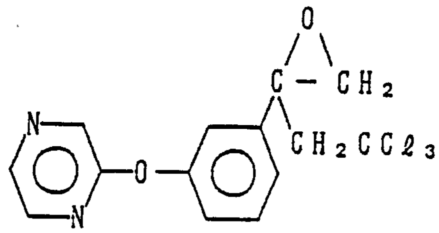

compound No. 37

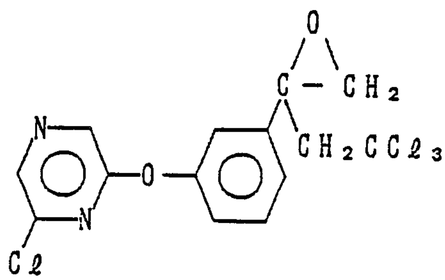

compound No. 38

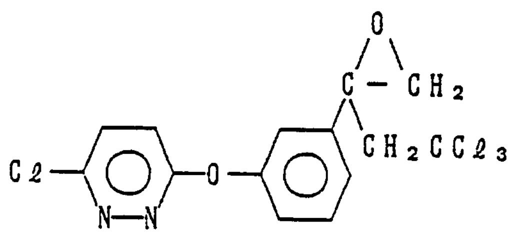

compound No. 39

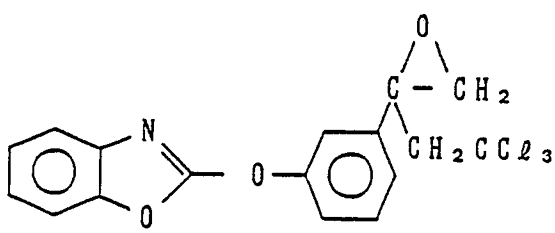

compound No. 40

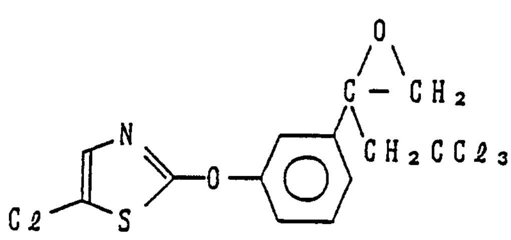

compound No. 41

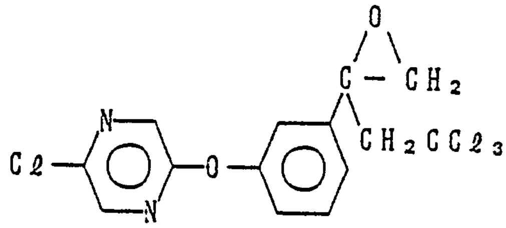

compound No. 42

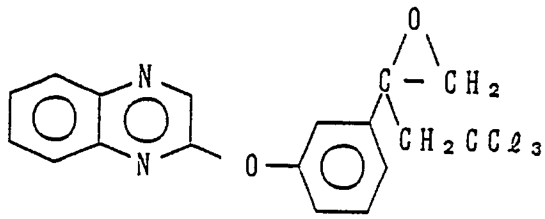

compound No. 43

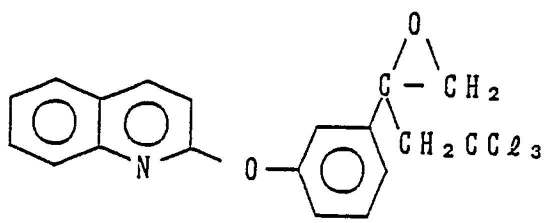

compound No. 44

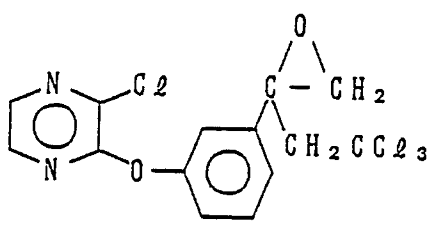

compound No. 45

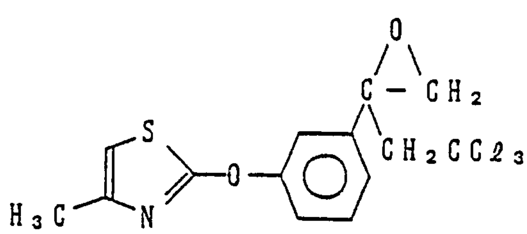

compound No. 46

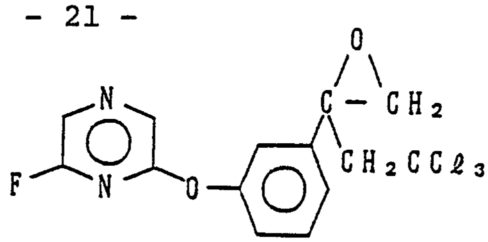

compound No. 47

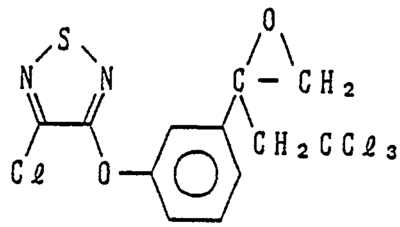

compound No. 48

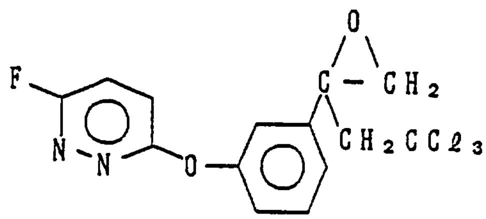

compound No. 49

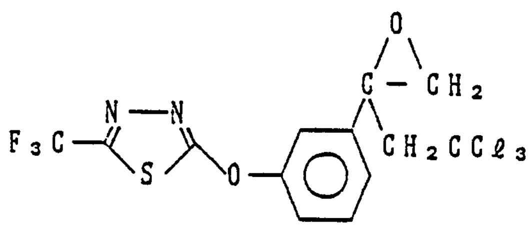

compound No. 50

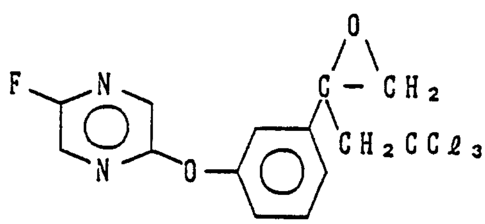

compound No. 51

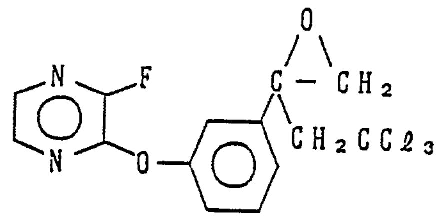

compound No. 52

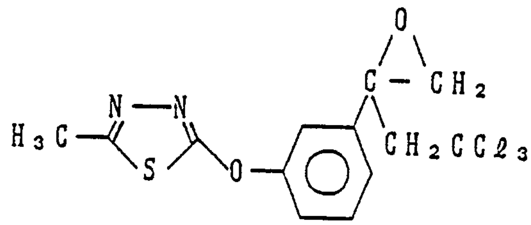

compound No. 53

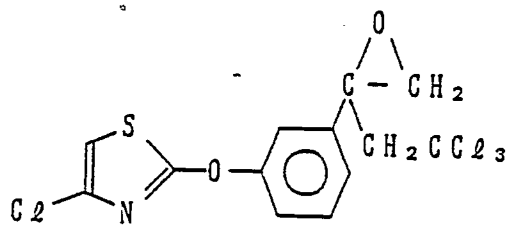

Further, the infrared absorption spectra and the nuclear magnetic resonance spectra thereof are shown in Table 1.

Table 1

| Compound No. | Infrared spectrum ($cm^{-1}$) | H-NMR spectrum 1) (ppm) |
|---|---|---|
| 1 | 2900~3100, 1610, 1590, 1330, 1130 | 2.90 (1H,d), 3.17 (1H,d), 3.37 (2H,s), 6.70~7.30 (5H,m), 7.83 (1H,dd), 8.35 (1H,brs) |
| 2 | 2900~3100, 1595, 1455, 1365, 1285, 1160 | 2.93 (1H,d), 3.15 (1H,d), 3.35 (2H,s), 6.80~7.90 (7H,m) |
| 3 | 2900~3150, 1595, 1320, 1150 | 2.97 (1H,d), 3.20 (1H,d), 3.40 (2H,s), 6.86 (1H,d), 7.00~7.40 (4H,m), 7.97 (1H,d) |
| 4 | 2900~3100, 1585, 1465, 1265 | 3.00 (1H,d), 3.20 (1H,d), 3.40 (2H,s), 6.87 (1H,d), 7.00~7.50 (5H,m), 7.65 (1H,dd), 8.11 (1H,d) |
| 5 | 2900~3100, 1595, 1415, 1250 | 3.00 (1H,d), 3.22 (1H,d), 3.40 (2H,s), 6.80~8.30 (8H,m) |
| 6 | 2900~3100, 1580, 1430, 1285 | 2.95 (1H,d), 3.20 (1H,d), 3.40 (2H,s), 6.60~7.70 (7H,m) |
| 7 | 2850~3150, 1570, 1535, 1290 | 2.95 (1H,d), 3.20 (1H,d), 3.38 (2H,s), 6.70~7.80 (7H,m) |
| 8 | 3000, 1610, 1580, 1440, 1240, 700 | 3.00 (1H,d), 3.19 (1H,d), 3.38 (2H,s), 3.76 (3H,s), 6.33 (1H,d), 6.45 (1H,d), 7.00~7.65 (5H,m) |
| 9 | 2970, 1580, 1460, 1370, 1270, 700 | 2.95 (1H,d), 3.17 (1H,d), 3.38 (2H,s), 6.81 (1H,d), 7.00~7.40 (4H,m), 7.75 (1H,dd), 8.16 (1H,d) |

Table 1 (Continued)

| Compound No. | Infrated spectrum ($cm^{-1}$) | H-NMR spectrum 1) (ppm) |
|---|---|---|
| 10 | 2940, 1580, 1450, 1230, 790, 700 | 2.44 (3H,s), 2.98 (1H,d), 3.20 (1H,d), 3.39 (2H,s), 6.60 (1H,d), 6.88 (1H,d), 7.00~7.70 (5H,m) |
| 11 | 3100, 1610, 1580, 1530, 1360, 1280 | 3.00 (1H,d), 3.26 (1H,d), 3.40 (2H,s), 7.05 (1H,d), 7.07~7.53 (4H,m), 8.50 (1H,dd), 9.00 (1H,d) |
| 12 | 3100, 1620, 1600, 1450, 1240, 1020, 800 | 2.99 (1H,d), 3.22 (1H,d), 3.40 (2H,s), 6.55~7.93 (7H,m) |
| 13 | 3000, 1600, 1580, 1440, 1240, 700 | 1.28 (3H,t), 2.99 (1H,d), 3.20 (1H,d), 3.39 (2H,s), 4.18 (2H,q), 6.41 (2H,t), 7.00~7.65 (5H,m) |
| 14 | 2960, 1600, 1500, 1280, 740 | 2.25 (3H,s), 2.96 (1H,d), 3.21 (1H,d), 3.39 (2H,s), 6.79 (1H,d), 7.10~7.59 (5H,m), 8.00 (1H,d) |
| 15 | 3100, 1480, 1440, 1240, 940 | 2.97 (1H,d), 3.21 (1H,d), 3.40 (2H,s), 7.00~7.63 (5H,m) |
| 16 | 2850~3100, 1600, 1335, 1140 | 2.92 (1H,d), 3.17 (1H,d), 3.37 (2H,s), 3.79 (3H,s), 6.55~7.05 (4H,m), 7.83 (1H,dd), 8.40 (1H,brs) |
| 17 | 2950, 1460, 1250, 1010, 920, 700 | 2.37 (3H,t), 2.97 (1H,d), 3.22 (1H,d), 3.40 (2H,s), 7.00~7.45 (4H,m) |
| 18 | 1415, 1350, 1190, 1150, 720 | 2.98 (1H,d), 3.22 (1H,d), 3.40 (2H,s), 7.07~7.52 (6H,m), 8.32 (1H,d) |

Table 1 (Continued)

| Compound No. | Infrared spectrum ($cm^{-1}$) | H-NMR spectrum 1) (ppm) |
|---|---|---|
| 19 | 3400, 3180, 1680, 1370, 1285 | 3.00 (1H, d), 3.23 (1H, d), 3.40 (2H, s), 6.40 (2H, brs), 6.91 (1H, d), 7.00~7.50 (4H, m), 8.15 (1H, d), 8.60 (1H, d) |
| 20 | 1590, 1435, 1265, 1055, 710 | 2.99 (1H, d), 3.22 (1H, d), 3.40 (2H, s), 6.96 (1H, dd), 7.05 ~7.50 (4H, m), 7.75 (1H, dd), 8.00 (1H, dd) |
| 21 | 3100, 2250, 1600, 1490, 1390, 1290, 710 | 2.98 (1H, d), 3.22 (1H, d), 3.40 (2H, s), 7.00 (1H, dd), 7.07 ~7.50 (4H, m), 7.94 (1H, dd), 8.55 (1H, dd) |
| 22 | 3100, 1595, 1440, 1320, 1280, 1135, 540 | 2.98 (1H, d), 3.01 (3H, s), 3.20 (1H, d), 3.40 (2H, s), 7.03~8.01 (7H, m) |
| 23 | 3100, 1580, 1430, 1290, 1095, 810, 710 | 2.96 (1H, d), 3.19 (1H, d), 3.39 (2H, s), 6.85~7.60 (6H, m), 8.19 (1H, t) |
| 24 | 1600, 1490, 1260, 710 | 2.95 (1H, d), 3.21 (1H, d), 3.40 (2H, s), 4.58 (2H, s), 6.90 (1H, d), 7.06~7.46 (4H, m), 7.75 (1H, dd), 8.17 (1H, d) |
| 25 | 1600, 1495, 1265, 940, 710 | 2.98 (1H, d), 3.20 (1H, d), 3.40 (2H, s), 5.31 (2H, d), 6.93 (1H, d), 7.00~7.47 (4H, m), 7.74 (1H, dt), 8.19 (1H, t) |
| 26 | 1595, 1490, 1360, 1265, 1085, 705 | 3.00 (1H, d), 3.23 (1H, d), 3.40 (2H, s), 6.66 (1H, t), 6.99 (1H, d), 7.07~7.56 (4H, m), 7.85 (1H, dd), 8.29 (1H, brd) |
| 27 | 3075, 1580, 1460, 1260, 705 | 2.96 (1H, d), 3.18 (1H, d), 3.37 (2H, s), 6.85~7.49 (6H, m), 8.11 (1H, m) |

Table 1 (Continued)

| Compound No. | Infrared spectrum ($cm^{-1}$) | H-NMR spectrum 1) (ppm) |
|---|---|---|
| 28 | 3300, 1680, 1490, 1400, 1260, 720 | 2.15 (3H, s), 2.95 (1H, d), 3.20 (1H, d), 3.39 (2H, s), 6.83 (1H, d), 6.90~7.65 (5H, m), 7.98~8.30 (2H, m) |
| 29 | 3100, 1530, 1480, 1240, 1170, 710 | 2.90 (1H, d), 3.21 (1H, d), 3.40 (1H, d), 3.70 (1H, d), 7.00~7.60 (6H, m) |
| 30 | 3100, 3000, 1540, 1455, 1240, 770 | 2.97 (1H, d), 3.20 (1H, d), 3.40 (2H, s), 7.20~7.81 (8H, m) |
| 31 | 2850~3050, 1570, 1400, 1300, 1240 | 3.00 (1H, d), 3.24 (1H, d), 3.40 (2H, s), 6.95~7.60 (5H, m), 8.54 (2H, d) |
| 32 | 3100, 1560, 1450, 1360, 970, 710 | 2.97 (1H, d), 3.20 (1H, d), 3.41 (2H, s), 6.90 (1H, s), 7.00~7.60 (4H, m), 8.55 (1H, s) |
| 33 | 3000, 1580, 1440, 1235, 1145, 970 | 2.97 (1H, d), 3.20 (1H, d), 3.40 (2H, s), 6.80 (1H, d), 7.04~7.60 (4H, m), 8.45 (1H, d) |
| 34 | 3070, 1570, 1420, 1320, 800, 710 | 3.00 (1H, d), 3.22 (1H, d), 3.40 (2H, s), 7.05~7.57 (4H, m), 8.56 (2H, s) |
| 35 | 2950, 1570, 1420, 1300, 710 | 2.26 (3H, s), 3.00 (1H, d), 3.25 (1H, d), 3.40 (2H, s), 7.10~7.50 (4H, m), 8.40 (2H, s) |
| 36 | 3095, 1590, 1410, 1290, 710 | 2.98 (1H, d), 3.23 (1H, d), 3.40 (2H, s), 7.05~7.60 (4H, m), 8.08 (1H, dd), 8.27 (1H, d), 8.41 (1H, d) |

Table 1 (Continued)

| Compound No. | Infrared spectrum ($cm^{-1}$) | H-NMR spectrum 1) (ppm) |
|---|---|---|
| 37 | 3000, 1410, 1300, 1175, 1010, 960, 710 | 2.96(1H,d), 3.19(1H,d), 3.40(2H,s), 7.00~7.60(4H,m), 8.28(2H,brs) |
| 38 | 3070, 1580, 1410, 1290, 700 | 2.93(1H,d), 3.20(1H,d), 3.42(2H,d), 7.05~7.62(6H,m) |
| 39 | 3100, 1520, 1410, 1200, 750 | 2.99(1H,d), 3.24(1H,d), 3.41(2H,s), 7.20~7.60(8H,m) |
| 40 | 1540, 1500, 1250, 1160, 715 | 2.96(1H,d), 3.21(1H,d), 3.40(2H,s), 7.05(1H,s), 7.15~7.50(4H,m) |
| 41 | 3100, 1540, 1415, 1305, 1180, 710 | 2.96(1H,d), 3.23(1H,d), 3.40(2H,s), 7.05~7.50(4H,m), 8.30(2H,s) |
| 42 | 3100, 1580, 1415, 1320, 1230, 780 | 3.00(1H,d), 3.20(1H,d), 3.40(2H,s), 7.18~8.17(8H,m), 8.66(1H,s) |
| 43 | 3100, 1600, 1440, 1320, 1245, 710 | 2.99(1H,d), 3.16(1H,d), 3.39(2H,s), 7.05(1H,d), 7.07~7.81(8H,m), 8.08 (1H,d) |
| 44 | 3080, 1400, 1180, 1100, 710 | 3.00(1H,d), 3.22(1H,d), 3.41(2H,s), 7.06~7.50(4H,m), 7.94(1H,d), 8.07 (1H,d) |
| 45 | 1520, 1500, 1250, 1100, 720 | 2.30(3H,d), 2.94(1H,d), 3.20(1H,d), 3.40(2H,s), 6.38(1H,s), 7.10~7.55 (4H,m) |

Table 1 (Continued)

| Compound No. | Infrared spectrum ($cm^{-1}$) | H-NMR spectrum 1) (ppm) |
|---|---|---|
| 46 | 1590, 1420, 1325, 1260, 1195, 710 | 3.00 (1H, d), 3.22 (1H, d), 3.40 (2H, s), 7.05~7.56 (4H, m), 8.13 (1H, d), 8.28 (1H, d) |
| 47 | 1510, 1410, 1185, 1110, 700 | 2.98 (1H, d), 3.25 (1H, d), 3.41 (2H, s), 7.28~7.57 (4H, m) |
| 48 | 3100, 1585, 1440, 1420, 1260, 715 | 2.97 (1H, d), 3.19 (1H, d), 3.40 (2H, s), 7.07~7.59 (6H, m) |
| 49 | 1480, 1340, 1170, 1045, 705 | 2.95 (1H, d), 3.21 (1H, d), 3.40 (2H, d), 7.24~7.60 (4H, m) |
| 50 | 1430, 1335, 1270, 1210, 710 | 3.00 (1H, d), 3.21 (1H, d), 3.40 (2H, d), 7.04~7.51 (4H, m), 8.14 (1H, d), 8.30 (1H, d) |
| 51 | 3100, 1440, 1400, 1200, 710 | 3.00 (1H, d), 3.22 (1H, d), 3.40 (2H, s), 7.03~7.49 (4H, m), 7.78~8.00 (2H, m) |
| 52 | 1490, 1450, 1270, 1210, 710 | 2.68 (3H, s), 2.95 (1H, d), 3.20 (1H, d), 3.40 (2H, s), 7.20~7.45 (4H, m) |
| 53 | 1520, 1500, 1250, 1110, 950, 720 | 2.94 (1H, d), 3.20 (1H, d), 3.40 (2H, s), 6.59 (1H, s), 7.17~7.68 (4H, m) |

1) Solvent: $CDCl_3$ Internal standard: tetramethyl silane

As is shown by the following equation, the oxirane derivative of the present invention can be prepared by the epoxidation of a butene derivative (IV) by using a peroxide.

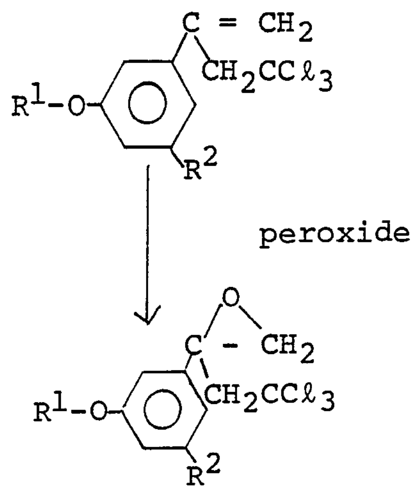

.... (IV)

.... (III)

($R^1$ and $R^2$ in the equation each have the same meaning as given before.)

The peroxide used in this reaction is not particularly limitative and any of those conventionally used in the epoxidation of an ethylenic double bond, such as cumene hydroperoxide, tert-butyl hydroperoxide, cyclohexyl hydroperoxide, organic percarboxylic acids, e.g., peracettic acid, α-hydroperoxy isobutyric acid, perbenzoic acid, m-chloro perbenzoic acid and the like, and so on, can be used. Among them, organic percarboxylic acids or, in particular, peracetic acid and m-chloro perbenzoic acid are preferred. This reaction is performed usually in an inert solvent, preferably, at a temperature in the range from 0 to 80°C. As the said inert solvent, for example, halogenated hydrocarbons such as methylene chloride, chloroform, carbon tetrachloride,

dichloroethane, 1,1,1-trichloroethane, o-dichlorobenzene and the like are used satisfactorily. If desired, the reaction can be performed by the admixture of the reaction mixture with a buffering agent such as sodium acetate, sodium benzoate and the like. The proportion of the above mentioned butene derivative (IV) and the peroxide to be used should desirably be such that the peroxide is used in a proportion of 1 to 3 times by equivalents relative to the butene derivative (IV). The reaction time depends on the reaction temperature, kind of the peroxide used and so on and cannot be given definitely but it is usually about 1 to 30 hours.

The oxirane derivative formed by the reaction can be recovered by a conventional method and, if necessary, may be purified by a known means such as column chromatography and the like.

The above mentioned butene derivative (IV) can be prepared in the following process from a propene derivative (V) as a starting material.

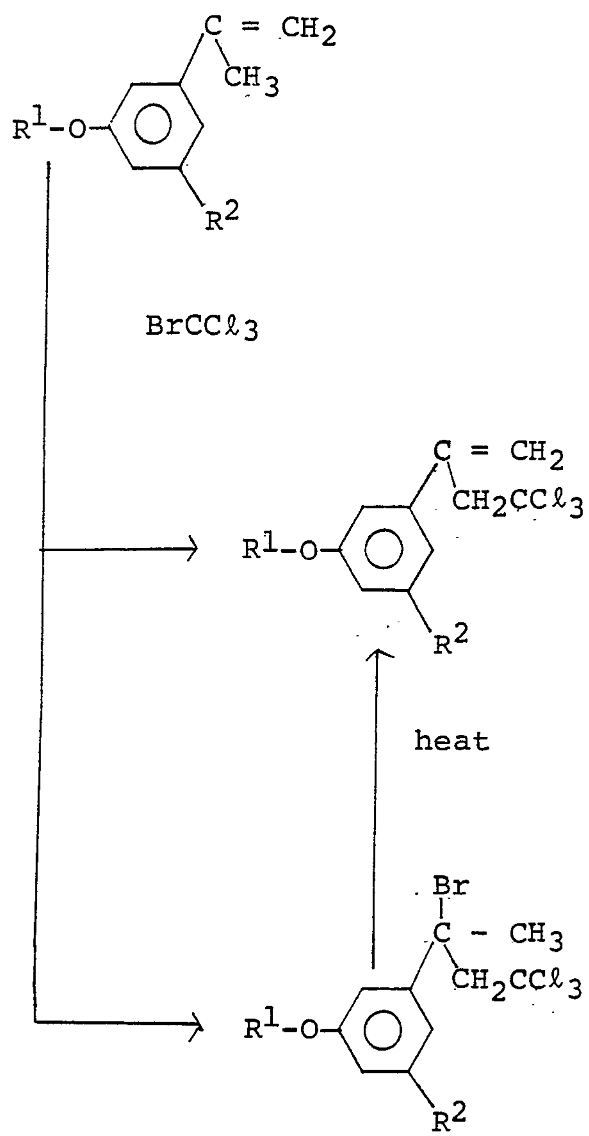


... (V)

... (IV)

... (VI)

($R^1$ and $R^2$ each have the same meaning as given before.) Namely, the desired butene derivative (IV) or bromo trichloro butane derivative (VI) can be obtained by reacting the propene derivative (V) and bromo trichloro methane in the presence of a catalyst such as copper (I) chloride and the like and an amine compound such as piperidine, cyclohexyl amine and the like, usually, at a temperature in the range from 0°C to the boiling point of the bromo trichloro methane for about 1 to 20 hours. It is optional in this case by adequately

selecting the reaction conditions that the butene derivative (IV) is directly derived or, alternatively, the bromo trichloro butane derivative (VI) is derived. When the bromo trichloro butane derivative (VI) is obtained, it can be converted into the desired butene derivative (IV) by heating the same in a solvent such as o-dichlorobenzene, 1,2-dichloro ethane and the like at a suitable temperature in the presence of a catalyst such as copper (I) chloride, iron (III) chloride and the like.

Further, the above mentioned propene derivative (V) can be prepared by the following two processes by using readily available starting materials.

<u>First process</u>

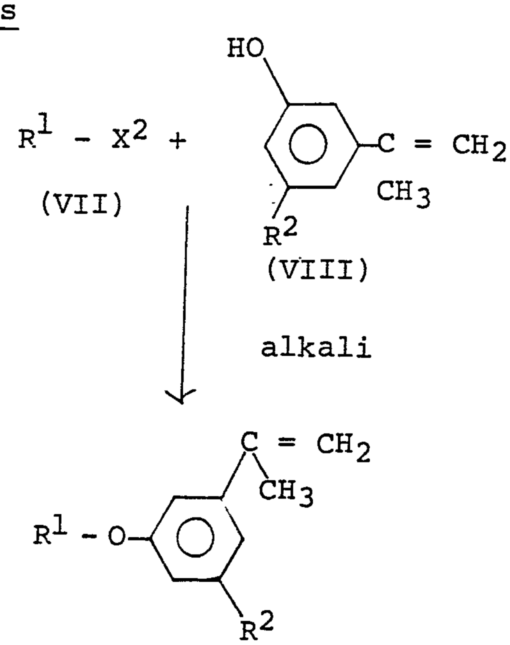


(VII) (VIII)

(V)

($R^1$ and $R^2$ each have the same meaning as given before and $X^2$ is a halogen atom).

Namely, the propene derivative (V) can be obtained by heating and reacting the nitrogen-containing aryl derivative (VII) and α-methyl styrene in the presence of an alkali, usually, at a temperature in the range from 0 to 120°C. This reaction is performed usually in a polar solvent or a solvent such as dimethyl formamide, dimethyl sulfoxide, N-methyl pyrrolidone and the like and potassium hydroxide, sodium hydroxide, potassium carbonate, sodium carbonate, potassium hydrogen carbonate, sodium hydrogen carbonate and the like can be used satisfactorily as the alkali.

Second process

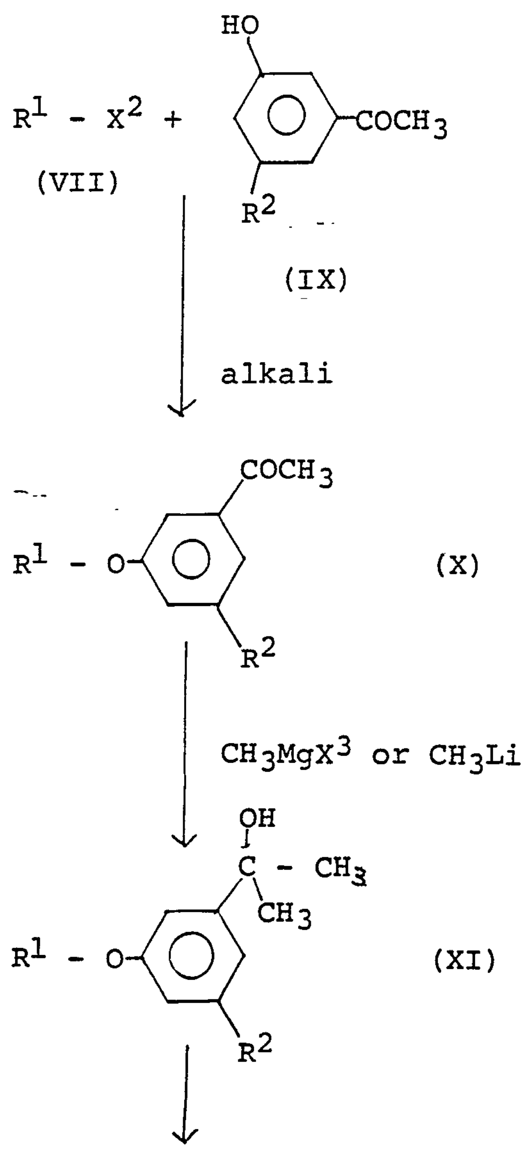


(VII)

(IX)

(X)

(XI)

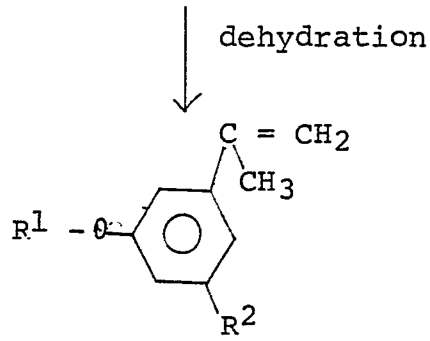


(V)

($R^1$ and $R^2$ each have the same meaning as given before and $X^2$ is a halogen atom.)

Namely, in the first place, the nitrogen-containing aryloxy acetophenone derivative (X) is obtained by heating and reacting the nitrogen-containing aryl derivative (VII) and the acetophenone derivative (IX) in the presence of an alkali, usually, at a temperature in the range from 0 to 120°C. This reaction is performed, usually, in a polar solvent or a solvent such as dimethyl formamide, dimethyl sulfoxide, N-methyl pyrrolidone and the like and potassium hydroxide, sodium hydroxide, potassium carbonate, sodium carbonate, potassium hydrogen carbonate, sodium hydrogen carbonate and the like can be used satisfactorily as the alkali.

In the next place, the nitrogen-containing aryloxy acetophenone derivative (X) is reacted in an ether such as diethyl ether, diisopropyl ether, tetrahydrofuran and the like with a Grignard reagent of methylmagnesium halide, methyl lithium and the like to give the carbinol compound (XI) and then this is heated at a temperature, usually, in the range from 100

to 150°C, preferably, in the presence of a polymerization inhibitor such as tert-butyl catechol and the like and an inorganic salt such as potassium sulfate and the like to effect dehydration so as to give the desired propene derivative (V).

The oxirane derivative of the present invention has excellent herbicidal activity against the annual weeds belonging to the family of Gramineae such as Echinochloa crus-galli P. Beauv. var. formosensis Ohwi and the like, annual weeds belonging to the family of Zingiberaceae such as Cyperus difformis L. and the like, annual broadleaf weeds such as Rotala indica (Willd.) Koehne var. uligirosa (Miq.) Koehne, Monochoria vaginalis Presl var. plantaginea and the like and perennial weeds such as Cyperus serotinus Rottb. and the like as the weeds in paddy fields along with high selectivity and these weeds can be effectively controlled with a dose of, for example, about 100 g per 10 ares without causing any chemical damages against paddy rice with the above mentioned dose.

The herbicide of the present invention contains the above mentioned oxirane derivative or, namely, the novel oxirane derivative of the present invention represented by the general formula (III) as an effective ingredient and can be used by preparing in the form of a water-dispersible powder, emulsion, dust, granules and the like by blending these compounds with a liquid carrier such as solvents and the like or a solid carrier such as fine mineral powders

and the like. In the preparation, a surface active agent can be added in order to impart emulsifiability, dispersibility, spreadability and the like.

When the herbicide of the present invention is used in the form of a water-dispersible powder, it is usual to use a composition prepared by compounding from 10 to 55% by weight of the oxirane derivative of the present invention, from 40 to 88% by weight of a solid carrier and from 2 to 5% by weight of a surface active agent. When it is used in the form of an emulsion, it is usual to prepare the same by compounding from 20 to 50% by weight of the oxirane derivative of the present invention, from 35 to 75% by weight of a solvent and from 5 to 15% by weight of a surface active agent.

When used in the form of a powder, on the other hand, it is usually prepared by compounding from 1 to 16% by weight of the oxirane derivative of the present invention, from 80 to 97% by weight of a solid carrier and from 2 to 5% by weight of a surface active agent. When used in the form of granules, the preparation is made by compounding from 1 to 15% by weight of the oxirane of the present invention, from 80 to 97% by weight of a solid carrier and from 2 to 5% by weight of a surface active agent. The solid carrier used here is a fine powder of a mineral material and the fine powder of a mineral material is exemplified by, for example, oxides such as diatomaceous earth, slaked lime and the like, phosphates such as apatite and the like, sulfates such as gypsum and the like,

silicates such as talc, pyrophyllite, clay, kaolin, bentonite, acid clay, fumed silica, quartz powder, silica stone powder and the like, and so on.

And, the solvent usable is an organic solvent which is particularly exemplified by aromatic hydrocarbons such as benzene, toluene, xylene and the like, chlorinated hydrocarbons such as o-chlorotoluene, trichloromethane, trichloroethylene and the like, alcohols such as cyclohexanol, amyl alcohol, ethylene glycol and the like, ketones such as isophorone, cyclohexanone, cyclohexenyl cyclohexanone and the like, ethers such as Butyl Cellosolve ®, dimethyl ether, methyl ethyl ether and the like, esters such as isopropyl acetate, benzyl acetate, methyl phthalate and the like and amides such as dimethyl formamide and the like as well as mixtures thereof.

As the surface active agent, furthermore, any of anionic, non-ionic, cationic and amphoteric (amino acids, betaine and the like) ones can be used.

It is optional that, according to need, the herbicide of the present invention contains, together with the oxirane derivative represented by the above given general formula (III), other herbicidally active ingredients. Such other herbicidally active ingredients are exemplified by hitherto known herbicides such as those of the phenoxy type, diphenyl ether type, triazine type, urea type, carbamate type, thiol carbamate type, acid anilide type, pyrazole type, phosphate

type, sulfonyl urea type, oxadiazone type and the like and these herbicides can be used as being adequately selected.

Further, the herbicide of the present invention can be used according to need by blending with insecticides, bactericides, plant growth controlling agents, fertilizers and the like.

[Examples]

In the following, the present invention is described in more detail by way of preparation examples and test examples although the present invention is never limited thereto.

Preparation Example 1

Step 1

5.58 g (0.02 mole) of 2-[3-(5-trifluoromethyl-2-pyridyloxy)phenyl]-1-propene were dissolved in 50 ml of bromo trichloro methane and 0.12 g of copper (I) chloride was added thereto followed by dropwise addition of 1.70 g (0.02 mole) of piperazine under chilling with ice and agitation was performed thereafter for 6 hours at room temperature. In the next place, it was admixed with a 5% by weight hydrochloric acid, extracted with chloroform and dried over anhydrous sodium sulfate. The crude product was purified by column chromatography (silica gel, ethyl acetate:hexane = 1:10) to give 6.69 g of 2-bromo-2-[3-(5-trifluoromethyl-2-pyridyloxy)-phenyl]-4,4,4-trichloro butane (yield 70%) in the form of a colorless oily matter.

Step 2

2.39 g (0.01 mole) of 2-bromo-2-[3-(5-trifluoromethyl-2-pyridyloxy)phenyl]-4,4,4-trichloro butane were dissolved in 50 ml of o-dichlorobenzene and, with addition of 1.43 g (0.01 mole) of copper (I) chloride, heated under reflux for 1 hour with agitation. After cooling, the o-dichlorobenzene layer was washed with water and dried over anhydrous sodium sulfate. The crude product was purified by column chromatography (silica gel, ethyl acetate:hexane = 1:10) to give 3.89 g (yield 70%) of 2-[3-(5-trifluoromethyl-2-pyridyloxy)-phenyl]-4,4,4-trichloro-1-butene in the form of a colorless oily matter.

Step 3

1.98 g (0.005 mole) of 2-[3-(5-trifluoromethyl-2-pyridyloxy)phenyl]-4,4,4-trichloro-1-butene were dissolved in 20 ml of methylene chloride to which a liquid prepared by dissolving 0.28 g of sodium acetate in 3.48 g of a 40% by weight aqueous solution of peracetic acid was added dropwise and agitated for 4 hours at room temperature. Further, the same amount of the sodium acetate/peracetic acid solution was added dropwise and agitated for 5 hours at 35 to 40°C. After cooling, the methylene chloride layer was washed with a saturated aqueous solution of sodium hydrogen carbonate and dried over anhydrous sodium sulfate. The crude product was purified by column chromatography (silica gel, ethyl acetate: hexane = 1:10) to give 1.58 g (Yield 75%) of 2-[3-(5-trifluoromethyl-2-pyridyloxy)phenyl]-2-(2,2,2-trichloroethyl)

oxirane in the form of a colorless oily matter. The analytical values and structural formula thereof are shown in Table 3.

Preparation Examples 2 and 3

The same procedure as in Preparation Example 1 was undertaken excepting the use of the substituted nitrogen-containing oxyphenyl propene in place of the 2-[3-(5-trifluoromethyl-2-pyridyloxy)phenyl]-1-propene to give the corresponding oxirane derivatives each in a yield shown in Table 2. The analytical values and structural formulas of these oxirane derivatives are shown in Table 3.

Preparation Example 4

Step 1

4.91 g (0.02 mole) of 2-[3-(5-chloro-2-pyridyloxy)-phenyl]-1-propene were dissolved in 50 ml of bromo trichloro methane and 0.12 g of copper (I) chloride was added thereto followed by dropwise addition of 1.70 g (0.02 mole) of piperidine and heating under reflux for 2 hours. In the next place, it was admixed with a 5% by weight hydrochloric acid, extracted with chloroform and dried over anhydrous sodium sulfate. The crude product was purified by column chromatography (silica gel, ethyl acetate:hexane = 1:10) to give 7.01 g (yield 97%) of 2-[3-(5-chloro-2-pyridyloxy)-phenyl]-4,4,4-trichloro-1-butene in the form of a colorless oily matter.

Step 2

3.62 g (0.01 mole) of 2-[3-(5-chloro-2-pyridyloxy)phenyl]-4,4,4-trichloro-1-butene were dissolved in 20 ml of methylene chloride and, after addition of 4.00 g (0.016 mole) of a 70% by weight m-chloro perbenzoic acid, agitated for 3 hours by heating at 40°C. In the next place, the methylene chloride solution was washed with a 20% by weight aqueous solution of sodium hydrogen sulfite, 5% by weight aqueous solution of potassium carbonate and saturated aqueous solution of sodium chloride and dried over anhydrous sodium sulfate. The crude product was purified by column chromatography (silica gel, ethyl acetate:hexane = 1:10) to give 1.41 g (yield 37%) of 2-[3-(5-chloro-2-pyridyloxy)phenyl]-2-(2,2,2-trichloroethyl) oxirane in the form of a colorless oily matter.

The analytical values and structural formula thereof are shown in Table 3.

Preparation Examples 5 to 53

The same procedure as in Preparation Example 4 was undertaken excepting the use of the substituted nitrogen-containing aryloxyphenyl propenes shown in Table 2 in place of the 2-[3-(5-chloro-2-pyridyloxy)phenyl]-1-propene in Preparation Example 4 to give the respective corresponding oxirane derivatives in the yield shown in Table 2. The analytical values and structural formulas of these oxirane derivatives are shown in Table 3.

Table 2

| Preparation Example | Phenyl propene as the starting material | Yield (%) | | |
|---|---|---|---|---|
| | | Step 1 | Step 2 | Step 3 |
| 2 | 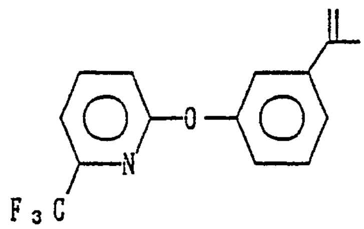 | 100 | 34 | 56 |
| 3 | 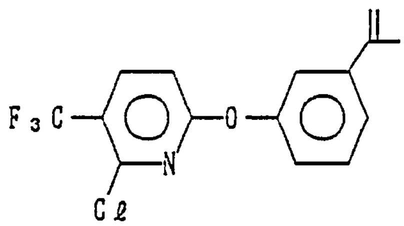 | 100 | 79 | 76 |
| 5 | 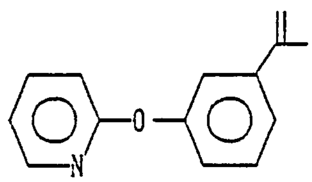 | 90 | 6 | – |
| 6 | 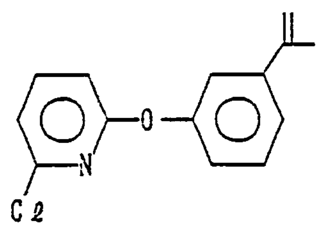 | 97 | 48 | – |
| 7 | 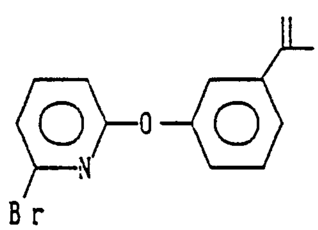 | 88 | 49 | – |

Table 2 (Continued)

| Preparation Example | Phenyl propene as the starting material | Yield (%) | | |
|---|---|---|---|---|
| | | Step 1 | Step 2 | Step 3 |
| 8 | 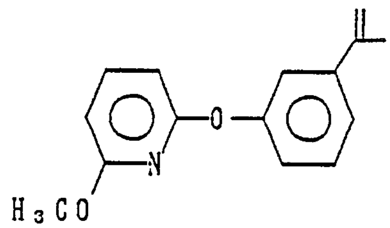 | 45 | 26 | – |
| 9 | 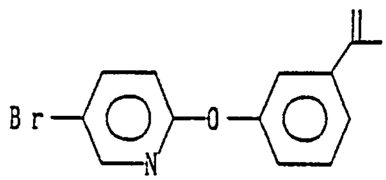 | 90 | 28 | – |
| 10 | 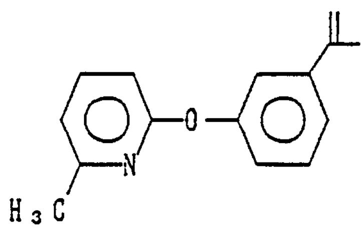 | 70 | 11 | – |
| 11 | 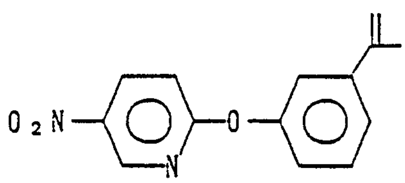 | 96 | 44 | – |
| 12 | 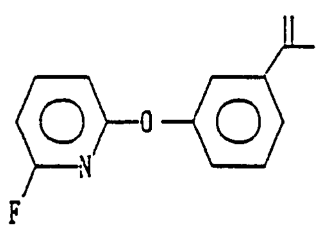 | 79 | 40 | – |

Table 2 (Continued)

| Preparation Example | Phenyl propene as the starting material | Yield (%) | | |
|---|---|---|---|---|
| | | Step 1 | Step 2 | Step 3 |
| 13 | 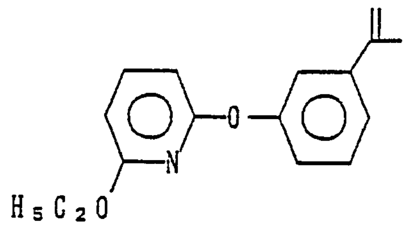 | 45 | 10 | – |
| 14 | 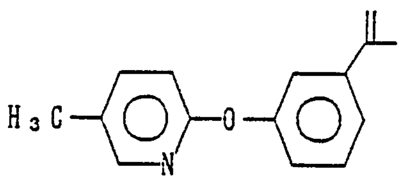 | 78 | 17 | – |
| 15 | 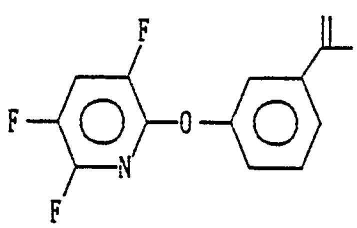 | 70 | 30 | – |
| 16 | 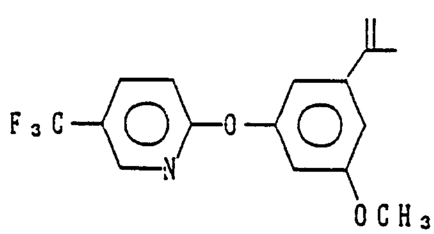 | 76 | 46 | – |

Table 2 (Continued)

| Preparation Example | Phenyl propene as the starting material | Yield (%) | |
|---|---|---|---|
| | | Step 1 | Step 2 |
| 17 | 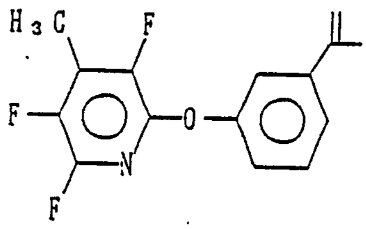 | 75 | 49 |
| 18 | 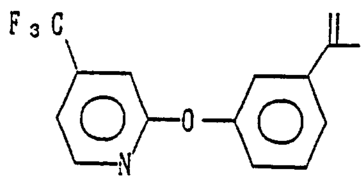 | 100 | 25 |
| 19 | 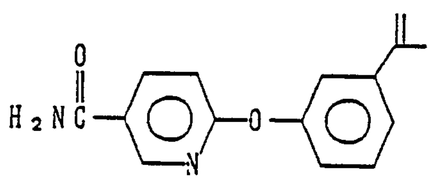 | 67 | 33 |
| 20 | 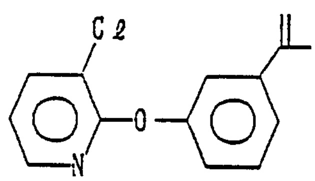 | 91 | 17 |
| 21 | 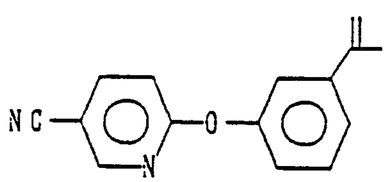 | 79 | 36 |
| 22 | 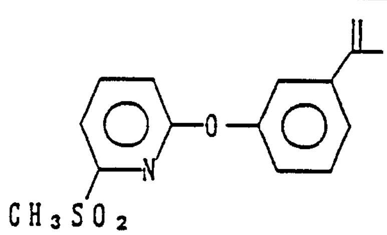 | 96 | 41 |

Table 2 (Continued)

| Preparation Example | Phenyl propene as the starting material | Yield (%) | |
|---|---|---|---|
| | | Step 1 | Step 2 |
| 23 | 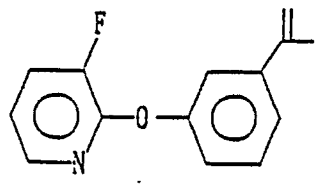 | 80 | 30 |
| 24 | 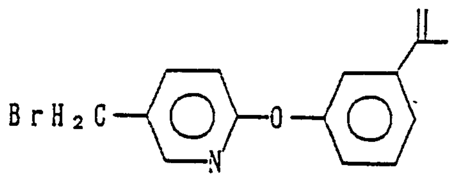 | 71 | 10 |
| 25 | 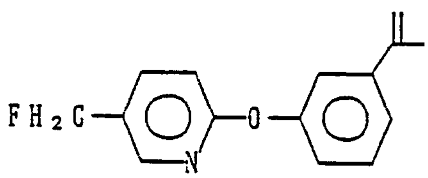 | 92 | 32 |
| 26 | 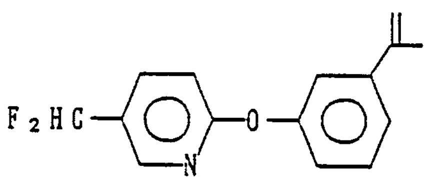 | 95 | 34 |
| 27 | 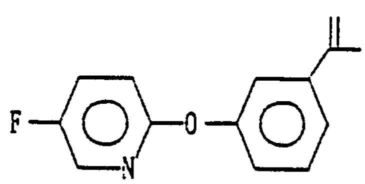 | 90 | 36 |
| 28 | 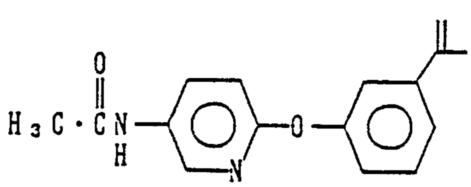 | 80 | 63 |

Table 2 (Continued)

| Preparation Example | Phenyl propene as the starting material | Yield (%) | |
|---|---|---|---|
| | | Step 1 | Step 2 |
| 29 | 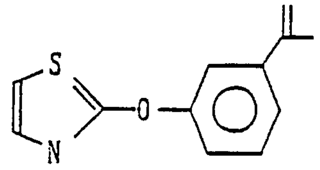 | 95 | 15 |
| 30 | 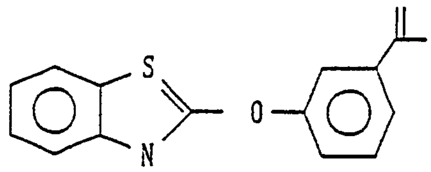 | 88 | 28 |
| 31 | 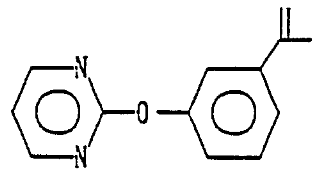 | 70 | 49 |
| 32 | 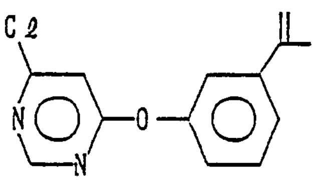 | 91 | 45 |
| 33 | 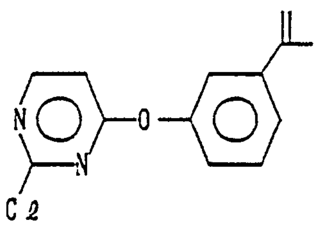 | 93 | 14 |
| 34 | 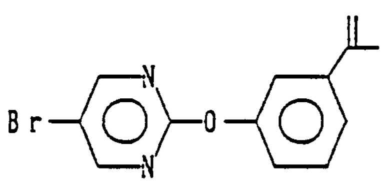 | 94 | 53 |

Table 2 (Continued)

| Preparation Example | Phenyl propene as the starting material | Yield (%) | |
|---|---|---|---|
| | | Step 1 | Step 2 |
| 35 | 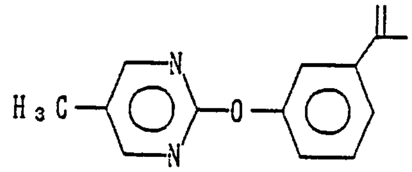 | 87 | 21 |
| 36 | 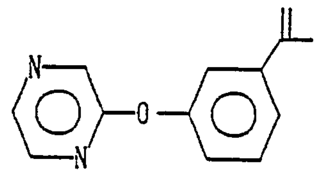 | 77 | 31 |
| 37 | 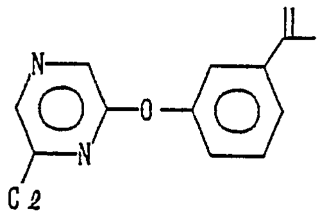 | 90 | 16 |
| 38 | 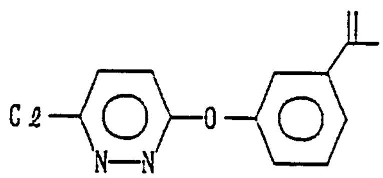 | 96 | 45 |
| 39 | 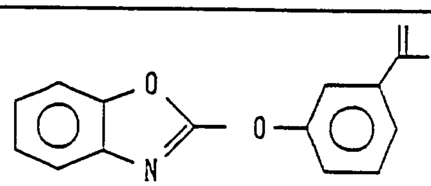 | 25 | 5 |
| 40 | 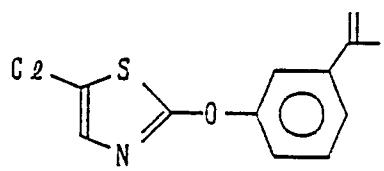 | 80 | 39 |
| 41 | 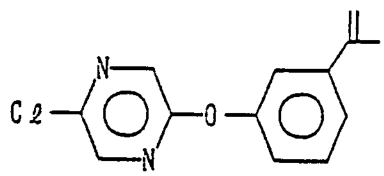 | 71 | 55 |

Table 2 (Continued)

| Preparation Example | Phenyl propene as the starting material | Yield (%) | |
|---|---|---|---|
| | | Step 1 | Step 2 |
| 42 | 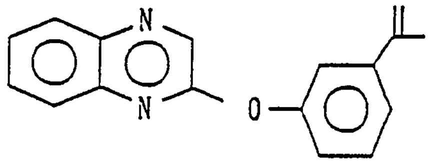 | 66 | 26 |
| 43 | 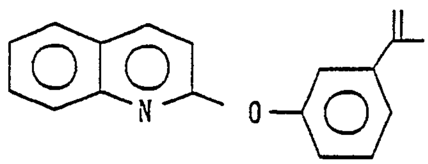 | 76 | 34 |
| 44 | 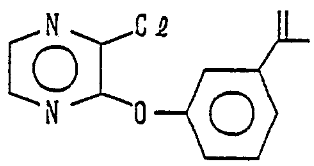 | 68 | 77 |
| 45 | 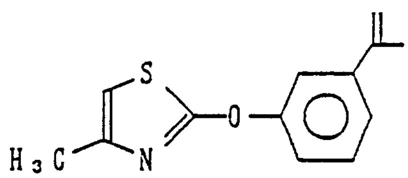 | 40 | 22 |
| 46 | 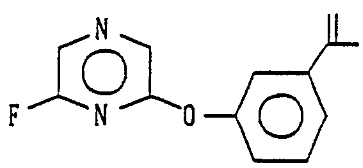 | 90 | 38 |
| 47 | 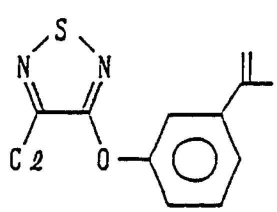 | 52 | 40 |

Table 2 (Continued)

| Preparation Example | Phenyl propene as the starting material | Yield (%) | |
|---|---|---|---|
| | | Step 1 | Step 2 |
| 48 | 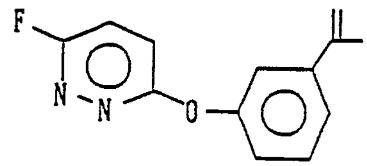 | 55 | 16 |
| 49 | 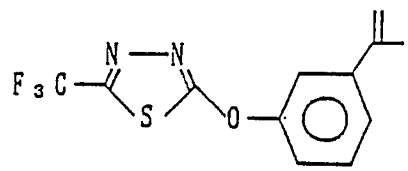 | 65 | 85 |
| 50 | 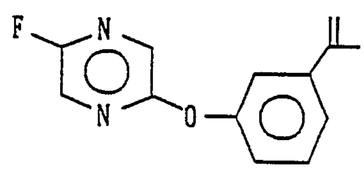 | 66 | 69 |
| 51 | 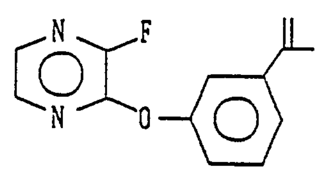 | 80 | 89 |
| 52 | 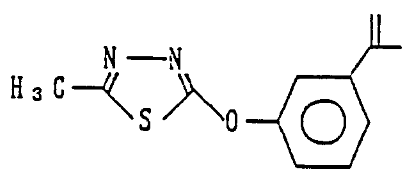 | 53 | 62 |
| 53 | 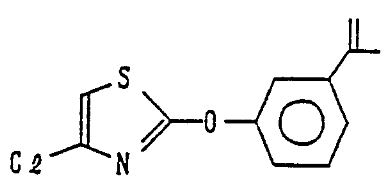 | 70 | 20 |

Table 3

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 1 Compound No. 1 | $C_{16}H_{11}NO_2Cl_3F_3$ 412.62 | 46.26 (46.57) | 2.71 (2.68) | 3.33 (3.39) | 2900~3100, 1610, 1590, 1330, 1130 | 2.90(1H,d), 3.17(1H,d), 3.37(2H,s), 6.70~7.30(5H,m), 7.83(1H,dd), 8.35 (1H,brs) |
| 2 Compound No. 2 | $C_{16}H_{11}NO_2Cl_3F_3$ 412.62 | 46.58 (46.57) | 2.81 (2.68) | 3.49 (3.39) | 2900~3100, 1595, 1455, 1365, 1285, 1160 | 2.93(1H,d), 3.15(1H,d), 3.35(2H,s), 6.80~7.90(7H,m) |
| 3 Compound No. 3 | $C_{16}H_{10}NO_2Cl_4F_3$ 447.07 | 42.74 (42.98) | 2.64 (2.25) | 3.30 (3.13) | 2900~3150, 1595, 1320, 1150 | 2.97(1H,d), 3.20(1H,d), 3.40(2H,s), 6.86(1H,d), 7.00~7.40(4H,m), 7.97 (1H,d) |
| 4 Compound No. 4 | $C_{15}H_{11}NO_2Cl_4$ 379.07 | 47.30 (47.52) | 2.99 (2.92) | 3.68 (3.69) | 2900~3100, 1585, 1465, 1265 | 3.00(1H,d), 3.20(1H,d), 3.40(2H,s), 6.87(1H,d), 7.00~7.50(5H,m), 7.65 (1H,dd), 8.11(1H,dd) |
| 5 Compound No. 5 | $C_{15}H_{12}NO_2Cl_3$ 344.62 | 52.10 (52.27) | 3.55 (3.50) | 4.19 (4.06) | 2900~3100, 1595, 1415, 1250 | 3.00(1H,d), 3.22(1H,d), 3.40(2H,s), 6.80~8.30(8H,m) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 6 Compound No. 6 | $C_{15}H_{11}NO_2Cl_4$ 397.07 | 47.39 (47.52) | 3.17 (2.92) | 3.94 (3.69) | 2900~3100, 1580, 1430, 1285 | 2.95 (1H, d), 3.20 (1H, d), 3.40 (2H, s), 6.60~7.70 (7H, m) |
| 7 Compound No. 7 | $C_{15}H_{11}NO_2BrCl_3$ 423.52 | 42.91 (42.53) | 2.30 (2.61) | 3.02 (3.30) | 2850~3150, 1570, 1535, 1290 | 2.95 (1H, d), 3.20 (1H, d), 3.38 (2H, s), 6.70~7.80 (7H, m) |
| 8 Compound No. 8 | $C_{16}H_{14}NO_3Cl_3$ 374.65 | 50.89 (51.29) | 3.83 (3.76) | 3.70 (3.73) | 3000, 1610, 1580, 1440, 1240, 700 | 3.00 (1H, d), 3.19 (1H, d), 3.38 (2H, s), 3.76 (3H, s), 6.33 (1H, d), 6.45 (1H, d), 7.00~7.65 (5H, m) |
| 9 Compound No. 9 | $C_{15}H_{11}NO_2BrCl_3$ 423.52 | 42.31 (42.53) | 2.80 (2.61) | 3.15 (3.30) | 2970, 1580, 1460, 1370, 1270, 700 | 2.95 (1H, d), 3.17 (1H, d), 3.38 (2H, s), 6.81 (1H, d), 7.00~7.40 (4H, m), 7.75 (1H, dd), 8.16 (1H, d) |
| 10 Compound No. 10 | $C_{16}H_{14}NO_2Cl_3$ 358.65 | 53.51 (53.58) | 4.10 (3.93) | 3.77 (3.90) | 2940, 1580, 1450, 1230, 790, 700 | 2.44 (3H, s), 2.98 (1H, d), 3.20 (1H, d), 3.39 (2H, s), 6.60 (1H, d), 6.88 (1H, d), 7.00~7.70 (5H, m) |

Table 3 (Continued)

| Preparation Example | Molecular formula and molecular weight | Results of analysis | | | | |
|---|---|---|---|---|---|---|
| | | Elementary analysis (%) [1] | | | Infrared spectrum ($cm^{-1}$) | H-NMR spectrum (ppm) [2] |
| | | C | H | N | | |
| 11 Compound No.11 | $C_{15}H_{11}N_2O_4Cl_3$ 389.62 | 46.04 (46.24) | 3.05 (2.84) | 7.15 (7.18) | 3100, 1610, 1580, 1530, 1360, 1280 | 3.00(1H,d), 3.26(1H,d), 3.40(2H,s), 7.05(1H,d), 7.07～7.53(4H,m), 8.50(1H,dd), 9.00(1H,d) |
| 12 Compound No.12 | $C_{15}H_{11}NO_2Cl_3F$ 362.61 | 49.53 (49.68) | 3.24 (3.05) | 3.86 (3.86) | 3100, 1620, 1600, 1450, 1240, 1020, 800 | 2.99(1H,d), 3.22(1H,d), 3.40(2H,s), 6.55～7.93(7H,m) |
| 13 Compound No.13 | $C_{17}H_{16}NO_3Cl_3$ 388.68 | 52.44 (52.53) | 4.19 (4.14) | 3.52 (3.60) | 3000, 1600, 1580, 1440, 1240, 700 | 1.28(3H,t), 2.99(1H,d), 3.20(1H,d), 3.39(2H,s), 4.18(2H,q), 6.41(2H,t), 7.00～7.65(5H,m) |
| 14 Compound No.14 | $C_{16}H_{14}NO_2Cl_3$ 358.65 | 53.01 (53.58) | 4.22 (3.93) | 3.69 (3.90) | 2960, 1600, 1500, 1280, 740 | 2.25(3H,s), 2.96(1H,d), 3.21(1H,d), 3.39(2H,s), 6.79(1H,d), 7.10～7.59(5H,m), 8.00(1H,d) |
| 15 Compound No.15 | $C_{15}H_9NO_2Cl_3F_3$ | 44.89 (45.19) | 2.56 (2.27) | 3.58 (3.51) | 3100, 1480, 1440, 1240, 940 | 2.97(1H,d), 3.21(1H,d), 3.40(2H,s), 7.00～7.63(5H,m) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 16 Compound No.16 | $C_{17}H_{13}NO_3Cl_3F_3$ 442.65 | 46.50 (46.12) | 3.03 (2.96) | 3.04 (3.16) | 2850～3100, 1600, 1335, 1140 | 2.92(1H,d), 3.17(1H,d), 3.37(2H,s), 3.79(3H,s), 6.55～7.05(4H,m), 7.83 (1H,dd), 8.40(1H,brs) |
| 17 Compound No.17 | $C_{16}H_{11}NO_2Cl_3F_3$ 412.62 | 46.51 (46.57) | 2.73 (2.68) | 3.20 (3.39) | 2950, 1460, 1250, 1010, 920, 700 | 2.37(3H,t), 2.79(1H,d), 3.22(1H,d), 3.40(2H,s), 7.00～7.45(4H,m) |
| 18 Compound No.18 | $C_{16}H_{11}NO_2Cl_3F_3$ 412.62 | 46.13 (46.57) | 2.89 (2.68) | 3.57 (3.39) | 1415, 1350, 1190, 1150, 720 | 2.98(1H,d), 3.22(1H,d), 3.40(2H,s), 7.07～7.52(6H,m), 8.32(1H,d) |
| 19 Compound No.19 | $C_{16}H_{13}N_2O_3Cl_3$ 387.65 | 49.57 (49.57) | 3.10 (3.38) | 7.01 (7.22) | 3400, 3180, 1680, 1370, 1285 | 3.00(1H,d), 3.23(1H,d), 3.40(2H,s), 6.40(2H,brs), 6.91(1H,d), 7.00～7.50 (4H,m), 8.15(1H,d), 8.60(1H,d) |
| 20 Compound No.20 | $C_{15}H_{11}NO_2Cl_4$ 379.07 | 47.20 (47.52) | 3.20 (2.92) | 3.39 (3.69) | 1590, 1435, 1265, 1055, 710 | 2.99(1H,d), 3.22(1H,d), 3.40(2H,s), 6.96(1H,dd), 7.05 ～7.50(4H,m), 7.75 (1H,dd), 8.00(1H,dd) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 21 Compound No.21 | $C_{16}H_{11}N_2O_2C\ell_3$ 369.63 | 52.00 (51.99) | 3.35 (2.99) | 7.19 (7.57) | 3100, 2250, 1600, 1490, 1390, 1290, 710 | 2.98(1H,d), 3.22(1H,d), 3.40(2H,s), 7.00(1H,dd), 7.07 ~7.50(4H,m), 7.94 (1H,dd), 8.55(1H,dd) |
| 22 Compound No.22 | $C_{16}H_{14}NO_4SC\ell_3$ 422.71 | 45.54 (45.46) | 3.36 (3.33) | 3.38 (3.31) | 3100, 1595, 1440, 1320, 1280, 1135, 540 | 2.98(1H,d), 3.01(3H,s), 3.20(1H,d), 3.40(2H,s), 7.03~8.01(7H,m) |
| 23 Compound No.23 | $C_{15}H_{11}NO_2C\ell_3F$ 362.61 | 49.27 (49.68) | 3.39 (3.05) | 3.75 (3.86) | 3100, 1580, 1430, 1290, 1095, 810, 710 | 2.96(1H,d), 3.19(1H,d), 3.39(2H,s), 6.85~7.60(6H,m), 8.19(1H,t) |
| 24 Compound No.24 | $C_{16}H_{13}NO_2BrC\ell_3$ 437.55 | 43.83 (43.92) | 3.04 (2.99) | 3.21 (3.20) | 1600, 1490, 1260, 710 | 2.95(1H,d), 3.21(1H,d), 3.40(2H,s), 4.58(2H,s), 6.90(1H,d), 7.06~7.46 (4H,m), 7.75(1H,dd), 8.17(1H,d) |

Table 3 (Continued)

| Preparation Example | Molecular formula and molecular weight | Results of analysis: Elementary analysis (%) 1) C | H | N | Infrared spectrum ($cm^{-1}$) | H-NMR spectrum (ppm) 2) |
|---|---|---|---|---|---|---|
| 25 Compound No.25 | $C_{16}H_{13}NO_2Cl_3F$ 376.64 | 50.88 (51.02) | 3.60 (3.47) | 3.74 (3.71) | 1600, 1495, 1265, 940, 710 | 2.98(1H,d), 3.20(1H,d), 3.40(2H,s), 5.31(2H,d), 6.93(1H,d), 7.00~7.47 (4H,m), 7.74(1H,dt), 8.19(1H,t) |
| 26 Compound No.26 | $C_{16}H_{12}NO_2Cl_3F_2$ 394.63 | 48.51 (48.69) | 3.00 (3.06) | 3.29 (3.54) | 1595, 1490, 1360, 1265, 1085, 705 | 3.00(1H,d), 3.23(1H,d), 3.40(2H,s), 6.66(1H,t), 6.99(1H,d), 7.07~7.56 (4H,m), 7.85(1H,dd), 8.29(1H,brd) |
| 27 Compound No.27 | $C_{15}H_{11}NO_2Cl_3F$ 362.61 | 49.33 (49.68) | 3.42 (3.05) | 3.57 (3.86) | 3075, 1580, 1460, 1260, 705 | 2.96(1H,d), 3.18(1H,d), 3.37(2H,s), 6.85~7.49(6H,m), 8.11(1H,m) |
| 28 Compound No.28 | $C_{17}H_{15}N_2O_3Cl_3$ 401.68 | 50.77 (50.83) | 3.55 (3.76) | 6.93 (6.97) | 3300, 1680, 1490, 1400, 1260, 720 | 2.15(3H,s), 2.95(1H,d), 3.20(1H,d), 3.39(2H,s), 6.83(1H,d), 6.90~7.65 (5H,m), 7.98~8.30(2H,m) |
| 29 Compound No.29 | $C_{13}H_{10}NO_2SCl_3$ 350.65 | 44.46 (44.53) | 3.09 (2.87) | 3.81 (3.99) | 3100, 1530, 1480, 1240, 1170, 710 | 2.90(1H,d), 3.21(1H,d), 3.40(1H,d), 3.70(1H,d), 7.00~7.60(6H,m) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 30 Compound No.30 | $C_{17}H_{12}NO_2SCl_3$ 400.71 | 50.77 (50.95) | 3.30 (3.01) | 3.25 (3.49) | 3100, 3000, 1540, 1455, 1240, 770 | 2.97(1H,d), 3.20(1H,d), 3.40(2H,s), 7.20~7.81(8H,m) |
| 31 Compound No.31 | $C_{14}H_{11}N_2O_2Cl_3$ 345.61 | 48.32 (48.65) | 3.51 (3.20) | 8.02 (8.10) | 2850~3050, 1570, 1400, 1300, 1240 | 3.00(1H,d), 3.24(1H,d), 3.40(2H,s), 6.95~7.60(5H,m), 8.54(2H,d) |
| 32 Compound No.32 | $C_{14}H_{10}N_2O_2Cl_4$ 380.06 | 44.30 (44.24) | 2.82 (2.65) | 7.38 (7.37) | 3100, 1560, 1450, 1360, 970, 710 | 2.97(1H,d), 3.20(1H,d), 3.41(2H,s), 6.90(1H,s), 7.00~7.60(4H,m), 8.55 (1H,s) |
| 33 Compound No.33 | $C_{14}H_{10}N_2O_2Cl_4$ 380.06 | 44.18 (44.24) | 2.91 (2.65) | 7.40 (7.37) | 3000, 1580, 1440, 1235, 1145, 970 | 2.97(1H,d), 3.20(1H,d), 3.40(2H,s), 6.80(1H,d), 7.04~7.60(4H,m), 8.45 (1H,d) |
| 34 Compound No.34 | $C_{14}H_{10}N_2O_2BrCl_3$ 424.51 | 39.53 (39.61) | 2.48 (2.37) | 6.60 (6.59) | 3070, 1570, 1420, 1320, 800, 710 | 3.00(1H,d), 3.22(1H,d), 3.40(2H,s), 7.05~7.57(4H,m), 8.56(2H,s) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 35 Compound No.35 | $C_{14}H_{13}N_2O_2Cl_3$ 347.63 | 48.51 (48.37) | 3.66 (3.76) | 8.11 (8.05) | 2950, 1570, 1420, 1300, 710 | 2.26(3H,s), 3.00(1H,d), 3.25(1H,d), 3.40(2H,s), 7.10～7.50(4H,m), 8.40 (2H,s) |
| 36 Compound No.36 | $C_{14}H_{11}N_2O_2Cl_3$ 345.61 | 48.22 (48.65) | 3.47 (3.20) | 8.05 (8.10) | 3095, 1590, 1410, 1290, 710 | 2.98(1H,d), 3.23(1H,d), 3.40(2H,s), 7.05～7.60(4H,m), 8.08(1H,dd), 8.27 (1H,d), 8.41(1H,d) |
| 37 Compound No.37 | $C_{14}H_{10}N_2O_2Cl_4$ 380.06 | 44.00 (44.24) | 2.95 (2.65) | 7.40 (7.37) | 3000, 1410, 1300, 1175, 1010, 960, 710 | 2.96(1H,d), 3.19(1H,d), 3.40(2H,s), 7.00～7.60(4H,m), 8.28(2H,brs) |
| 38 Compound No.38 | $C_{14}H_{10}N_2O_2Cl_4$ 380.06 | 44.05 (44.24) | 2.86 (2.65) | 7.21 (7.37) | 3070, 1580, 1410, 1290, 700 | 2.93(1H,d), 3.20(1H,d), 3.42(2H,d), 7.05～7.62(6H,m) |
| 39 Compound No.39 | $C_{17}H_{12}NO_3Cl_3$ 384.65 | 52.79 (53.08) | 3.40 (3.14) | 3.33 (3.64) | 3100, 1520, 1410, 1200, 750 | 2.99(1H,d), 3.24(1H,d), 3.41(2H,s), 7.20～7.60(8H,m) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 40 Compound No.40 | $C_{13}H_{9}NO_{2}SCl_{4}$ 385.09 | 40.57 (40.54) | 2.51 (2.35) | 3.88 (3.63) | 1540, 1500, 1250, 1160, 715 | 2.96(1H,d), 3.21(1H,d), 3.40(2H,s), 7.05(1H,s), 7.15～7.50(4H,m) |
| 41 Compound No.41 | $C_{14}H_{10}N_{2}O_{2}Cl_{4}$ 380.06 | 43.79 (44.24) | 2.85 (2.65) | 7.55 (7.37) | 3100, 1540, 1415, 1305, 1180, 710 | 2.96(1H,d), 3.23(1H,d), 3.40(2H,s), 7.05～7.50(4H,m), 8.30(2H,s) |
| 42 Compound No.42 | $C_{18}H_{13}N_{2}O_{2}Cl_{3}$ 395.67 | 54.51 (54.64) | 3.50 (3.31) | 7.06 (7.07) | 3100, 1580, 1415, 1320, 1230, 780 | 3.00(1H,d), 3.20(1H,d), 3.40(2H,s), 7.18～8.17(8H,m), 8.66(1H,s) |
| 43 Compound No.43 | $C_{19}H_{14}NO_{2}Cl_{3}$ 394.68 | 57.66 (57.82) | 3.39 (3.57) | 3.51 (3.54) | 3100, 1600, 1440, 1320, 1245, 710 | 2.99(1H,d), 3.16(1H,d), 3.39(2H,s), 7.05(1H,d), 7.07～7.81(8H,m), 8.08 (1H,d) |
| 44 Compound No.44 | $C_{14}H_{10}N_{2}O_{2}Cl_{4}$ 380.06 | 44.19 (44.24) | 2.70 (2.65) | 7.34 (7.37) | 3080, 1400, 1180, 1100, 710 | 3.00(1H,d), 3.22(1H,d), 3.41(2H,s), 7.06～7.50(4H,m), 7.94(1H,d), 8.07 (1H,d) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 45 Compound No.45 | $C_{14}H_{12}NO_2SCl_3$ 364.67 | 45.99 (46.11) | 3.50 (3.31) | 3.81 (3.84) | 1520, 1500, 1250, 1100, 720 | 2.30(3H,d), 2.94(1H,d), 3.20(1H,d), 3.40(2H,s), 6.38(1H,s), 7.10~7.55 (4H,m) |
| 46 Compound No.46 | $C_{14}H_{10}N_2O_2Cl_3F$ 363.60 | 46.21 (46.24) | 2.80 (2.77) | 7.77 (7.70) | 1590, 1420, 1325, 1260, 1195, 710 | 3.00(1H,d), 3.22(1H,d), 3.40(2H,s), 7.05~7.56(4H,m), 8.13(1H,d), 8.28 (1H,d) |
| 47 Compound No.47 | $C_{12}H_8N_2O_2SCl_4$ 386.08 | 37.04 (37.33) | 2.32 (2.08) | 7.22 (7.25) | 1510, 1410, 1185, 1110, 700 | 2.98(1H,d), 3.25(1H,d), 3.41(2H,s), 7.28~7.57(4H,m) |
| 48 Compound No.48 | $C_{14}H_{10}N_2O_2Cl_3F$ 363.60 | 46.03 (46.24) | 2.93 (2.77) | 7.66 (7.70) | 3100, 1585, 1440, 1420, 1260, 715 | 2.97(1H,d), 3.19(1H,d), 3.40(2H,s), 7.07~7.59(6H,m) |
| 49 Compound No.49 | $C_{13}H_8N_2O_2SCl_3F_3$ 419.63 | 36.94 (37.20) | 1.99 (1.92) | 6.41 (6.67) | 1480, 1340, 1170, 1045, 705 | 2.95(1H,d), 3.21(1H,d), 3.40(2H,d), 7.24~7.60(4H,m) |

Table 3 (Continued)

| Preparation Example | Molecular formula and molecular weight | Results of analysis | | | | |
|---|---|---|---|---|---|---|
| | | Elementary analysis (%) 1) | | | Infrared spectrum ($cm^{-1}$) | H-NMR spectrum (ppm) 2) |
| | | C | H | N | | |
| 50 Compound No.50 | $C_{14}H_{10}N_2O_2Cl_3F$ 363.60 | 46.24 (46.24) | 2.80 (2.77) | 7.68 (7.70) | 1430, 1335, 1270, 1210, 710 | 3.00(1H,d), 3.21(1H,d), 3.40(2H,d), 7.04～7.51(4H,m), 8.14(1H,d), 8.30 (1H,d) |
| 51 Compound No.51 | $C_{14}H_{10}N_2O_2Cl_3F$ 363.60 | 45.98 (46.24) | 2.92 (2.77) | 7.65 (7.70) | 3100, 1440, 1400, 1200, 710 | 3.00(1H,d), 3.22(1H,d), 3.40(2H,s), 7.03～7.49(4H,m), 7.78～8.00(2H,m) |
| 52 Compound No.52 | $C_{13}H_{11}N_2O_2SCl_3$ 365.66 | 42.77 (42.70) | 3.15 (3.03) | 7.73 (7.66) | 1490, 1450, 1270, 1210, 710 | 2.68(3H,s), 2.95(1H,d), 3.20(1H,d), 3.40(2H,s), 7.20～7.45(4H,m) |
| 53 Compound No.53 | $C_{13}H_9NO_2SCl_4$ 385.09 | 40.29 (40.54) | 2.51 (2.35) | 3.37 (3.63) | 1520, 1500, 1250, 1110, 950, 720 | 2.94(1H,d), 3.20(1H,d), 3.40(2H,s), 6.59(1H,s), 7.17～7.68(4H,m) |

1) The value in branckets shows the theoretical value.

2) Solvent: $CDCl_3$, Internal standard: tetramethyl silane

Test Examples 1 to 53

(1) Preparation of herbicide

A carrier for water-dispersible powdery preparation was obtained by uniformly pulverizing and mixing 97 parts by weight of talc (name of commercial product: Sieglite) as the carrier, 1.5 parts by weight of an alkyl aryl sulfonic acid salt (name of commercial product: Neoperex, manufactured by Kao Atlas Co.) as a surface active agent and 1.5 parts by weight of a nonionic type and anionic type surface active agent (name of commerical product: Solpol 800A, manufactured by Toho Chemical Industry Co.).

A herbicide was obtained by uniformly pulverizing and mixing 90 parts by weight of this carrier for water-dispersible powder and 10 parts by weight of the oxirane derivative obtained in the above described Preparation Examples 1 to 53.

(2) Biological test (treatment test of water-filled soil)

A 1/15500 are porcelain-made pot was filled with paddy field soil and seeds of Echinochloa crus-galli P. Beauv. var. formosensis Ohwi, Cyperus difformis L., broadleaf weeds Rotala indica (Willd.) Koehne var. uligirosa (Miq.) Koehne and Monochoria vaginalis Presl var. plantaginea and Scirpus juncoides Roxb. ssp. Hotarui (Ohwi) T. Koyama were evenly sown to the suraface layer thereof, a tuber of Cyperus serotinus Rottb. was transplanted and paddy rice at the bifoliate period was transplanted.

Thereafter, a specified volume of a diluted liquid of

the herbicide obtained in (1) described above was evenly dripped to the water surface to effect treatment before germination of the weeds and the pot was thereafter kept standing in a greenhouse with water-sprinkling at appropriate intervals.

Twenty days after the treatment with the liquid agent, the herbicidal effect and the chemical damage against the rice crop were examined to give the results shown in Table 4. Meanwhile, the dose is shown by the amount of the effective ingredient per 10 ares. The chemical damage against paddy rice and the herbicidal effect are shown in the following manner by the measurement of the respective air-dried weights.

| Degree of chemical damage | Chemical damage against paddy rice (relative to control zone) |
|---|---|
| 0 | 100% |
| 1 | 95 to 99% |
| 2 | 90 to 94% |
| 3 | 80 to 89% |
| 4 | 60 to 79% |
| 5 | 50 to 59% |

| Degree of herbicidal effect | Herbicidal effect (relative to control zone) |
|---|---|
| 0 | 100% |
| 1 | 61 to 99% |
| 2 | 21 to 60% |
| 3 | 11 to 20% |
| 4 | 1 to 10% |
| 5 | 0% |

Comparative Test Example 1

The same procedure as in Test Example 1 was undertaken excepting the use of 2-(3,5-dichlorophenyl)-2-(2,2,2-trichloroethyl) oxirane [A] (trivial name: tridiphane, official publication of Japanese Patent Publication 53-3749) expressed by the structural formula

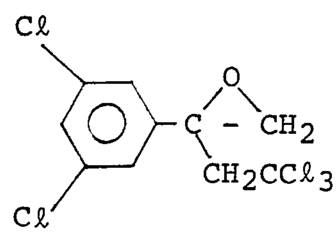

in place of the oxirane derivative obtained in Preparation Example 1, in Test Example 1. The results are shown in Table 4.

Comparative Test Example 2

The same procedure as in Test Example 1 was undertaken excepting the use of 2-(3-benzyloxyphenyl)-2-(2,2,2-trichloroethyl) oxirane [B] (specifications of U.S. Patents No. 4,103,772 and No. 4,018,801) expressed by the structural formula

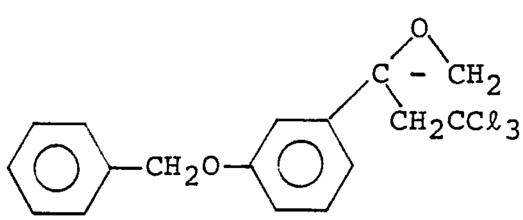

in place of the oxirane derivative obtained in Preparation Example 1, in Test Example 1. The results are shown in Table 4.

Table 4

| Test Example | Kind of the oxirane derivative | Amount of herbicide (g/10ares) | Herbicidal effect | | | | | Phytotoxicity to the paddy rice plants |
|---|---|---|---|---|---|---|---|---|
| | | | Echinochloa crus-galli P. Beauv. var. formosensis Ohwi | Cyperus serotinus Rottb. | Scirpus juncoides Roxb. ssp. Hotarui (Ohwi) T. Koyama | Cyperus difformis L. | Annual broadleaf weeds | |
| 1 | Compound of Preparation Example 1 | 100 | 5 | 5 | 3 | 5 | 5 | 0 |
| 2 | " 2 | 100 | 5 | 5 | 3 | 5 | 5 | 0 |
| 3 | " 3 | 100 | 5 | 5 | 3 | 5 | 5 | 0 |
| 4 | " 4 | 100 | 5 | 5 | 3 | 5 | 5 | 0 |
| 5 | " 5 | 100 | 5 | 5 | 3 | 5 | 5 | 0 |
| 6 | " 6 | 100 | 5 | 5 | 3 | 5 | 5 | 0 |
| 7 | " 7 | 100 | 5 | 5 | 3 | 5 | 5 | 0 |
| 8 | " 8 | 100 | 5 | 5 | 3 | 5 | 5 | 0 |
| 9 | " 9 | 100 | 5 | 5 | 3 | 5 | 5 | 0 |
| 10 | " 10 | 100 | 5 | 5 | 3 | 5 | 5 | 0 |
| 11 | " 11 | 100 | 5 | 5 | 3 | 5 | 5 | 0 |
| 12 | " 12 | 100 | 5 | 5 | 3 | 5 | 5 | 0 |
| 13 | " 13 | 100 | 5 | 5 | 3 | 5 | 5 | 0 |
| 14 | " 14 | 100 | 5 | 5 | 3 | 5 | 5 | 0 |
| 15 | " 15 | 100 | 5 | 5 | 3 | 5 | 5 | 0 |
| 16 | " 16 | 100 | 5 | 5 | 3 | 5 | 5 | 0 |
| 17 | " 17 | 100 | 5 | 5 | 3 | 5 | 5 | 0 |
| 18 | " 18 | 100 | 5 | 5 | 3 | 5 | 5 | 0 |
| 19 | " 19 | 100 | 5 | 5 | 3 | 5 | 5 | 0 |
| 20 | " 20 | 100 | 5 | 5 | 3 | 5 | 5 | 0 |

Table 4 (Continued)

| Test Example | Kind of the oxirane derivative | Amount of herbicide (g/10ares) | Herbicidal effect | | | | | Phytotoxicity to the paddy rice plants |
|---|---|---|---|---|---|---|---|---|
| | | | Echinochloa crus-galli P. Beauv. var. formosensis Ohwi | Cyperus serotinus Rottb. | Scirpus juncoides Roxb. ssp. Hotarui (Ohwi) T. Koyama | Cyperus difformis L. | Annual broadleaf weeds | |
| 21 | Compound of Preparation Example 21 | 100 | 5 | 5 | 3 | 5 | 5 | 0 |
| 22 | " 22 | 100 | 5 | 5 | 3 | 5 | 5 | 0 |
| 23 | " 22 | 100 | 5 | 5 | 3 | 5 | 5 | 0 |
| 24 | " 24 | 100 | 5 | 5 | 3 | 5 | 5 | 0 |
| 25 | " 25 | 100 | 5 | 5 | 3 | 5 | 5 | 0 |
| 26 | " 26 | 100 | 5 | 5 | 3 | 5 | 5 | 0 |
| 27 | " 27 | 100 | 5 | 5 | 3 | 5 | 5 | 0 |
| 28 | " 28 | 100 | 5 | 4 | 3 | 5 | 4 | 0 |
| 29 | " 29 | 100 | 5 | 5 | 3 | 5 | 4 | 0 |
| 30 | " 30 | 100 | 5 | 3 | 3 | 4 | 5 | 0 |
| 31 | " 31 | 100 | 5 | 5 | 3 | 5 | 5 | 0 |
| 32 | " 32 | 100 | 5 | 5 | 3 | 5 | 5 | 0 |
| 33 | " 33 | 100 | 5 | 3 | 3 | 5 | 5 | 0 |
| 34 | " 34 | 100 | 5 | 5 | 3 | 5 | 5 | 0 |
| 35 | " 35 | 100 | 5 | 5 | 3 | 5 | 5 | 0 |
| 36 | " 36 | 100 | 5 | 5 | 3 | 5 | 5 | 0 |
| 37 | " 37 | 100 | 5 | 5 | 3 | 5 | 5 | 0 |
| 38 | " 38 | 100 | 5 | 5 | 3 | 5 | 5 | 0 |
| 39 | " 39 | 100 | 5 | 5 | 3 | 5 | 5 | 0 |
| 40 | " 40 | 100 | 5 | 5 | 3 | 5 | 5 | 0 |

Table 4 (Continued)

| Test Example | Kind of the oxirane derivative | Amount of herbicide (g/10ares) | Herbicidal effect: Echinochloa crus-galli P. Beauv. var. formosensis Ohwi | Herbicidal effect: Cyperus serotinus Rottb. | Herbicidal effect: Scirpus juncoides Roxb. ssp. Hotarui (Ohwi) T. Koyama | Herbicidal effect: Cyperus difformis L. | Herbicidal effect: Annual broadleaf weeds | Phytotoxicity to the paddy rice plants |
|---|---|---|---|---|---|---|---|---|
| 41 | Compound of Preparation Example 41 | 100 | 5 | 5 | 3 | 5 | 5 | 0 |
| 42 | " 42 | 100 | 5 | 4 | 3 | 5 | 5 | 0 |
| 43 | " 43 | 100 | 5 | 5 | 3 | 5 | 4 | 0 |
| 44 | " 44 | 100 | 5 | 5 | 3 | 5 | 5 | 0 |
| 45 | " 45 | 100 | 5 | 5 | 4 | 5 | 5 | 0 |
| 46 | " 46 | 100 | 5 | 5 | 4 | 5 | 5 | 0 |
| 47 | " 47 | 100 | 5 | 5 | 3 | 5 | 5 | 0 |
| 48 | " 48 | 100 | 5 | 3 | 3 | 5 | 5 | 0 |
| 49 | " 49 | 100 | 5 | 5 | 4 | 5 | 5 | 0 |
| 50 | " 50 | 100 | 5 | 5 | 3 | 5 | 5 | 0 |
| 51 | " 51 | 100 | 5 | 5 | 3 | 5 | 5 | 0 |
| 52 | " 52 | 100 | 5 | 5 | 3 | 5 | 5 | 0 |
| 53 | " 53 | 100 | 5 | 5 | 4 | 5 | 5 | 0 |
| Comparative Example 1 | [A] | 100 | 5 | 5 | 0 | 5 | 4 | 4 |
| " 2 | [B] | 100 | 3 | 1 | 0 | 3 | 2 | 0 |

Possibility of Industrial Application

The oxirane derivative of the present invention is a novel compound having a specific nitrogen-containing aryloxyphenyl skeleton capable of being prepared by the disclosed method and it has excellent herbicidal activity against weeds in paddy fields along with excellent selectivity or, in particular, inter-genus selectivity between paddy rice and Echinochloa crus-galli P. Beauv. var. formosensis Ohwi so that it is useful as a herbicide for paddy rice. Weeds in paddy fields can be efficiently removed with the herbicide of the present invention at a low dose and no chemical damage is caused to paddy rice with the dose.

CLAIMS

1. An oxirane derivative represented by the general formula

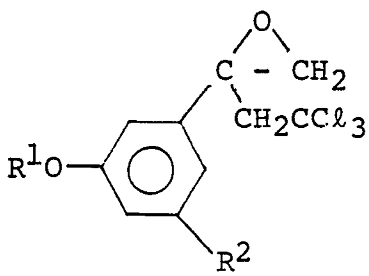

(in the formula, $R^1$ is

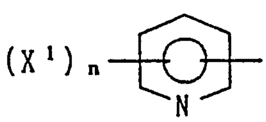 , 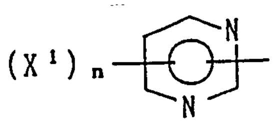 , 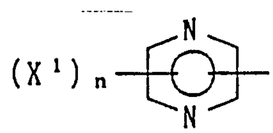 ,

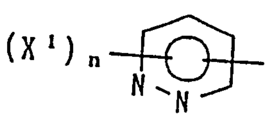 , 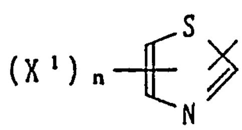 , 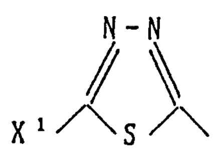 ,

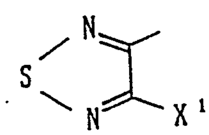 , 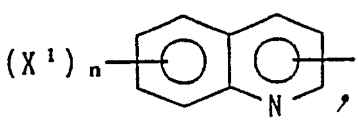 , 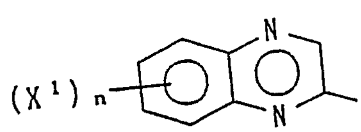 ,

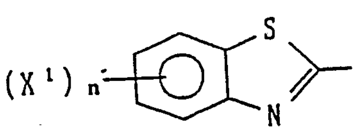 or 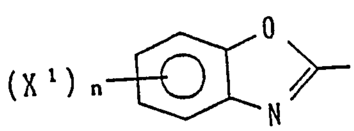 ,

$X^1$ is a halogen atom, alkyl group having 1 to 4 carbon atoms, haloalkyl group having 1 to 4 carbon atoms, alkoxy group having 1 to 4 carbon atoms, nitro group, cyano group, acid amide group, acylamino group having 1 to 4 carbon atoms or alkylsulfonyl group having 1 to 4 carbon atoms, n is an integer of 0 to 4 and $R^2$ is a hydrogen atom or an alkoxy

group having 1 to 4 carbon atoms, each $X^1$ being the same as or different from the others when n is a plural number).

2. A herbicide containing, as an effective ingredient, an oxirane derivative represented by the general formula

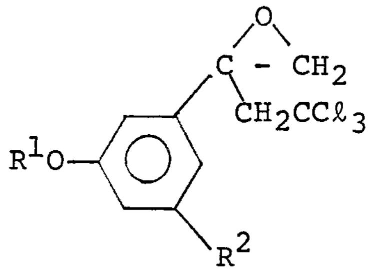

(in the formula, $R^1$ is

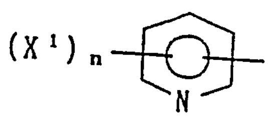 , 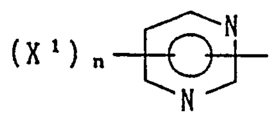 , 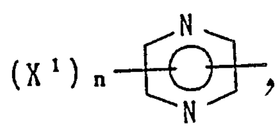 ,

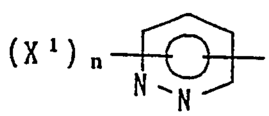 , 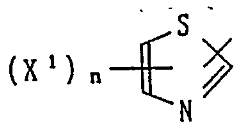 , 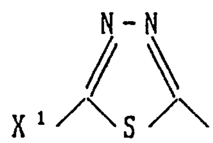 ,

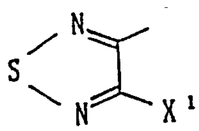 , 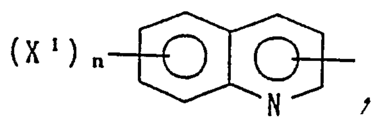 , 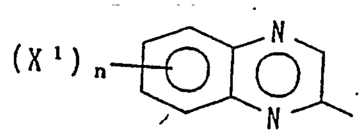 ,

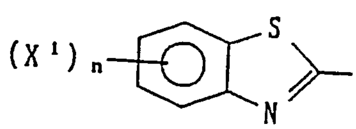 or 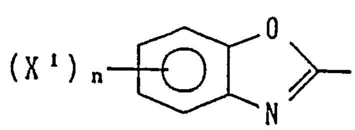 ,

$X^1$ is a halogen atom, alkyl group having 1 to 4 carbon atoms, haloalkyl group having 1 to 4 carbon atoms, alkoxy group having 1 to 4 carbon atoms, nitro group, cyano group, acid amide group, acylamino group having 1 to 4 carbon atoms or alkylsulfonyl group having 1 to 4 carbon atoms, n is an

integer of 0 to 4 and $R^2$ is a hydrogen atom or an alkoxy group having 1 to 4 carbon atoms, each $X^1$ being the same as or different from the others when n is a plural number).

# INTERNATIONAL SEARCH REPORT

International Application No PCT/JP89/00847

**I. CLASSIFICATION OF SUBJECT MATTER** (if several classification symbols apply, indicate all) [6]

According to International Patent Classification (IPC) or to both National Classification and IPC

Int. $Cl^4$ C07D405/12, 413/12, 417/12

**II. FIELDS SEARCHED**

Minimum Documentation Searched [7]

| Classification System | Classification Symbols |
|---|---|
| IPC | C07D405/12, 413/12, 417/12 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| | | |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| October 23, 1989 (23. 10. 89) | November 6, 1989 (06. 11. 89) |
| **International Searching Authority** | **Signature of Authorized Officer** |
| Japanese Patent Office | |